# EUROPEAN PATENT APPLICATION

(11) **EP 4 814 013 A1**
(43) Date of publication of application: **30.09.2026**
(21) Application number: 24893237.8
(22) Date of filing: 06.11.2024
(51) Int. Cl.: C07K 16/28, C07K 16/46, C12N 15/13, C12N 15/85, C12N 15/70, C12N 5/10, C12N 1/21, A61K 39/395, A61P 35/00, C12R 1/19

(54) **ANTI-CD16A SINGLE-DOMAIN ANTIBODY AND USE THEREOF**

(30) Priority: 21.11.2023 CN 202311556133
(71) Applicant: Nanjing Regenecore Biotech Co., Ltd., Nanjing, Jiangsu 210000 (CN)
(72) Inventor: SU, Zhipeng, Nanjing, Jiangsu 210000 (CN); WANG, Yang, Nanjing, Jiangsu 210000 (CN); XIE, Wei, Nanjing, Jiangsu 210000 (CN)
(74) Representative: Ghirardi, Valeria
(86) International application number: PCT/CN2024/130111
(87) International publication number: WO 2025/108079

(57) **Abstract**

Provided are an anti-CD16a single-domain antibody and a use thereof, relating to the field of immunology. The single-domain antibody is composed of heavy chains, and the heavy chains include a heavy chain CDR1 shown in any one of SEQ ID NO: 11 to SEQ ID NO: 13, a heavy chain CDR2 shown in any one of SEQ ID NO: 14 to SEQ ID NO: 17, and a heavy chain CDR3 shown in any one of SEQ ID NO: 18 to SEQ ID NO: 21. The present invention has the beneficial effects that biological genetic engineering technology is used to screen out a single-domain antibody specific to CD16a, and the antibody has good affinity.

## Description

### FIELD

The present disclosure relates to a single-domain antibody capable of specifically binding to CD16a (hereinafter abbreviated as "CD16a single-domain antibody"), a pharmaceutical composition comprising the single-domain antibody as an effective ingredient, and a medicinal use thereof.

### BACKGROUND

CD16a is a class of low-affinity, dominant activating transmembrane receptors expressed on NK cells, macrophages, and mast cells, belonging to transmembrane receptor members of the immunoglobulin superfamily. On NK cells, the α chain of FcγRIIIA binds to an immunoreceptor tyrosine-based activation motif (ITAM) containing the FcεRIγ chain and/or the T cell receptor (TCR)/CD3ζ chain for signal transduction.

An antibody with one domain or a single-domain antibody (sdAb) is the smallest antibody molecule at present, with a molecular weight of 1/10 that of an intact antibody. In addition to the antigen reactivity of an intact antibody, a single-domain antibody has some unique functional properties, such as small molecular weight, high stability, excellent solubility, ease of expression, weak immunogenicity, strong penetrability, strong targeting ability, and low production cost, which almost perfectly overcomes the defects of traditional antibodies, such as extended development cycle, relatively low stability, and stringent storage conditions.

Therefore, it is particularly necessary to develop a CD16a antibody with a small molecular weight and good tumor permeability, which can be used for free assembly into a bispecific or multispecific antibody, and which also has a single variable domain (hereinafter also referred to as "single-domain") with high affinity.

### SUMMARY

The purpose of the present disclosure is to provide a single-domain antibody capable of specifically binding to CD16a and uses thereof.

A first aspect of the present invention provides an anti-CD16a single-domain antibody, which is composed of a heavy chain, comprising a heavy chain CDR1 as set forth in any one of SEQ ID NO: 11-SEQ ID NO: 13, a heavy chain CDR2 as set forth in any one of SEQ ID NO: 14-SEQ ID NO: 17, and a heavy chain CDR3 as set forth in any one of SEQ ID NO: 18-SEQ ID NO: 21.

Preferably, the amino acid sequences of the heavy chain CDR1, heavy chain CDR2, and heavy chain CDR3 are one of the following (1)-(5):
(1) the CDR1 as set forth in SEQ ID NO: 12, the CDR2 as set forth in SEQ ID NO: 17, and the CDR3 as set forth in SEQ ID NO: 21;
(2) the CDR1 as set forth in SEQ ID NO: 12, the CDR2 as set forth in SEQ ID NO: 14, and the CDR3 as set forth in SEQ ID NO: 21;
(3) the CDR1 as set forth in SEQ ID NO: 11, the CDR2 as set forth in SEQ ID NO: 16, and the CDR3 as set forth in SEQ ID NO: 20;
(4) the CDR1 as set forth in SEQ ID NO: 11, the CDR2 as set forth in SEQ ID NO: 16, and the CDR3 as set forth in SEQ ID NO: 19; and
(5) the CDR1 as set forth in SEQ ID NO: 13, the CDR2 as set forth in SEQ ID NO: 15, and the CDR3 as set forth in SEQ ID NO: 18.

The above CDR combinations (1)-(5) correspond to single-domain antibodies 23D1, 14A3, 4B5, 15E2, and 13G6, respectively.

All of the above sequences can be replaced with a sequence having "at least 80% homology" to the original sequence or a sequence with only one or a few amino acid substitutions; the homology is preferably "at least 85% homology", more preferably "at least 90% homology", more preferably "at least 95% homology", most preferably "at least 98% homology".

In one embodiment, in any one or more CDRs of the heavy chain CDR1, CDR2, and CDR3, any one to five amino acid residues may be respectively substituted with a conservative amino acid(s) thereof. Specifically, in the heavy chain CDR1, 1 to 5 amino acid residues may be substituted with a conservative amino acid(s) thereof; in the heavy chain CDR2, 1 to 5 amino acid residues may be substituted with a conservative amino acid(s) thereof; and in the heavy chain CDR3, 1 to 5 amino acid residues may be substituted with a conservative amino acid(s) thereof.

As used herein, the term "sequence homology" refers to the degree to which two (nucleotide or amino acid) sequences in an alignment have the same residues at the same positions, and is usually expressed as a percentage. Preferably, homology is determined over the entire length of the sequences being aligned. Therefore, two copies with identical sequences share 100% homology.

In some embodiments, the purpose of the invention can also be achieved by sequences with only one or a few amino acids substituted, such as those containing 1, 2, 3, 4, 5, 6, 7, 8, 9, or 10 conservative amino acid substitutions, with respect to the foregoing sequences. These forms of variation include (but are not limited to): deletion, insertion, and/or substitution of one or more (typically 1-50, preferably 1-30, more preferably 1-20, or most preferably 1-10) amino acids, and addition of one or more (typically at most 20, preferably at most 10, or more preferably at most 5) amino acids at the C-terminal and/or N-terminal. In fact, when determining the degree of sequence homology between two amino acid sequences or when identifying the combination of CDR1, CDR2 and CDR3 in a single-domain antibody, one skilled in the art may contemplate the so-called "conservative" amino acid substitution, and in the case of substitution, the substitution will preferably be a conservative amino acid substitution. The conservative amino acid substitution is generally described as an amino acid substitution in which an amino acid residue is replaced by another amino acid residue with a similar chemical structure, and this substitution has little or substantially no effect on the function, activity or other biological properties of the polypeptide. The conservative amino acid substitutions are common in the art, for example, a conservative amino acid substitution means the substitution of one or a few amino acids within the following groups (a)-(d) by another one or few amino acids within the same group: (a) polar negatively-charged residues and uncharged amides thereof: Asp, Asn, Glu, and Gln; (b) polar positively-charged residues: His, Arg, and Lys; (c) aromatic residues: Phe, Trp, and Tyr; and (d) aliphatic nonpolar or weakly-polar residues: Ala, Ser, Thr, Gly, Pro, Met, Leu, Ile, Val, and Cys. The following are particularly preferred conservative amino acid substitutions: Asp is substituted by Glu; Asn is substituted by Gln or His; Glu is substituted by Asp; Gln is substituted by Asn; His is substituted by Asn or Gln; Arg is substituted by Lys; Lys is substituted by Arg or Gln; Phe is substituted by Met, Leu, or Tyr; Trp is substituted by Tyr; Tyr is substituted by Phe or Trp; Ala is substituted by Gly or Ser; Ser is substituted by Thr; Thr is substituted by Ser; Gly is substituted by Ala or Pro; Met is substituted by Leu, Tyr, or Ile; Leu is substituted by Ile or Val; Ile is substituted by Leu or Val; Val is substituted by Ile or Leu; and Cys is substituted by Ser. Furthermore, those skilled in the art will be aware that the inventiveness of a single-domain antibody is embodied in the CDR1-3 regions, whereas the framework region sequences FR1-4 are not unchangeable, and the sequences of FR1-4 can be conservative sequence variants of the sequences disclosed in the present disclosure.

The meaning of "anti-CD16a single-domain antibody" in the present invention includes not only an intact single-domain antibody, but also fragments, derivatives, and analogs of the anti-CD16a single-domain antibody. As used herein, the terms "fragment", "derivative" and "analog" have the same meaning, and all refer to a polypeptide that substantially retains the same biological function or activity as the antibody of this invention. The polypeptide fragment, derivative, or analog of the present invention can be (i) a polypeptide in which one or more conservative or non-conservative amino acid residues (preferably conservative amino acid residues) are substituted, where such resultant substituted amino acid residues may or may not be encoded by genetic codes, or (ii) a polypeptide having a substituent group in one or more amino acid residues, or (iii) a polypeptide formed by fusion of a mature polypeptide with another compound (such as a compound that extends the half-life of the polypeptide, for example polyethylene glycol), or (iv) a polypeptide formed by fusion of an additional amino acid sequence to this polypeptide sequence (such as a leader sequence or secretory sequence, or a sequence used to purify this polypeptide or a proprotein sequence, or a fusion protein formed with an Fc tag). Such fragments, derivatives and analogs fall within the scope publicly known to those skilled in the art in light of the teachings herein.

In a preferred embodiment, the antibody sequence further comprises framework region FRs; the framework region FRs comprise the amino acid sequences of a FR1, a FR2, a FR3 and a FR4; and the amino acid sequences of the framework region FRs are, respectively, as follows:
the FR1 as set forth in any one of SEQ ID NOs: 22-26 or an FR1 variant, wherein the FR1 variant comprises at most 5 amino acid substitutions in the FR1;
the FR2 as set forth in any one of SEQ ID NOs: 27-29 or an FR2 variant, wherein the FR2 variant comprises at most 5 amino acid substitutions in the FR2;
the FR3 as set forth in any one of SEQ ID NOs: 30-34 or an FR3 variant, wherein the FR3 variant comprises at most 5 amino acid substitutions in the FR3; and
the FR4 as set forth in SEQ ID NO: 35 or an FR4 variant, wherein the FR4 variant comprises at most 5 amino acid substitutions in the FR4.

A second aspect of the present invention is to provide amino acid sequences of a single-domain antibody capable of binding to CD16a, wherein the amino acid sequences of the single-domain antibody are set forth in SEQ ID NOs: 1-5, respectively, or the single-domain antibody has at least 80% sequence homology with the amino acid sequence as set forth in any one of SEQ ID NOs: 1-5, and is capable of specifically binding to the CD16a protein, or the amino acid sequence of the single-domain antibody has at least one amino acid residue in the FR1, FR2, FR3 or FR4 sequence replaced by a conservative amino acid compared with any one of SEQ ID NOs: 1-5.

In one embodiment, the anti-CD16a single-domain antibody has at least 90%, 91%, 92%, 93%, 94%, 95%, 96%, 97%, 98%, 99%, or 100% sequence homology with an amino acid sequence selected from the group consisting of SEQ ID NOs: 1-5, and is capable of specifically binding to the CD16a protein.

A third aspect of the present invention is to provide an Fc-fusion antibody or a humanized antibody of any one of the aforementioned anti-CD16a single-domain antibodies.

A fourth aspect of the present invention is to provide a recombinant protein comprising any one of the aforementioned anti-CD16a single-domain antibodies.

A fifth aspect of the present invention is to provide a bispecific or multispecific antibody comprising the single-domain antibody according to any one of the foregoing, wherein the single-domain antibody serves as a first antigen-binding moiety that specifically binds to CD16a.

In an embodiment, the bispecific antibody or multispecific antibody further comprises a binding moiety specific for a different tumor antigen other than CD16a;
preferably, the different tumor antigen other than CD16a includes FOLR1, Her2, DEC205, CLEC9A, CEACAM5, CTLA4, CD3, CD7, CD11c, CD19, CD20, CD22, CD40, CD44, CD206, EGFR, EGFRvIII, Fibroblast activation protein (FAP), CA9, MMP-2, PD-L1, SIRPa, Trop2, GPC1, GPC3, cMET, VEGFR, Claudin18.2, Nkp46, CD30, NKG2D, IL-2Rβ, BCMA, CD123, TGF-β, CD38, IL-7, IL-8, FRα, NCR3, IL-15, Muc1, IL-16 or any other tumor antigen.

In an embodiment, the bispecific antibody includes, but is not limited to, bispecific antibodies against FOLR1/CD16a, Her2/CD16a, DEC205/CD16a, CLEC9A/CD16a, CEACAM5/CD16a, CTLA4/CD16a, CD3/CD16a, CD7/CD16a, CD11c/CD16a, CD19/CD16a, CD20/CD16a, CD22/CD16a, CD40/CD16a, CD44/CD16a, CD206/CD16a, EGFR/CD16a, EGFRvIII/CD16a, Fibroblast activation protein (FAP)/CD16a, CA9/CD16a, MMP-2/CD16a, PD-L1/CD16a, SIRPa/CD16a, Trop2/CD16a, GPC1/CD16a, GPC3/CD16a, cMET/CD16a, VEGFR/CD16a, Claudin18.2/CD16a, CD30/CD16a, NKG2D/CD16a, IL-2Rβ/CD16a, BCMA/CD16a, CD123/CD16a, TGF-β/CD16a, CD38/CD16a, IL-7/CD16a, IL-8/CD16a, FRα/CD16a, NCR3/CD16a, IL-15/CD16a, Muc1/CD16a, or IL-16/CD16a, or the like.

In one embodiment, the aforementioned multispecific antibody is a trispecific antibody, comprising a first antigen-binding moiety that specifically binds to CD16a, a second antigen-binding moiety that specifically binds to Nkp46, and a third antigen-binding moiety that specifically binds to a different tumor antigen other than CD16a and Nkp46.

In one embodiment, the trispecific antibody includes, but is not limited to, trispecific antibodies against FOLR1/CD16a/Nkp46, Her2/CD16a/Nkp46, DEC205/CD16a/Nkp46, CLEC9A/CD16a/Nkp46, CEACAM5/CD16a/Nkp46, CTLA4/CD16a/Nkp46, CD3/CD16a/Nkp46, CD7/CD16a/Nkp46, CD11c/CD16a/Nkp46, CD19/CD16a/Nkp46, CD20/CD16a/Nkp46, CD22/CD16a/Nkp46, CD40/CD16a/Nkp46, CD44/CD16a/Nkp46, CD206/CD16a/Nkp46, EGFR/CD16a/Nkp46, EGFRvIII/CD16a/Nkp46, Fibroblast activation protein(FAP)/CD16a/Nkp46, CA9/CD16a/Nkp46, MMP-2/CD16a/Nkp46, PD-L1/CD16a/Nkp46, SIRPa/CD16a/Nkp46, Trop2/CD16a/Nkp46, GPC1/CD16a/Nkp46, GPC3/CD16a/Nkp46, cMET/CD16a/Nkp46, VEGFR/CD16a/Nkp46, Claudin18.2/CD16a/Nkp46, CD30/CD16a/Nkp46, NKG2D/CD16a/Nkp46, IL-2Rβ/CD16a/Nkp46, BCMA/CD16a/Nkp46, CD123/CD16a/Nkp46, TGF-β/CD16a/Nkp46, CD38/CD16a/Nkp46, IL-7/CD16a/Nkp46, IL-8/CD16a/Nkp46, FRα/CD16a/Nkp46, NCR3/CD16a/Nkp46, IL-15/CD16a/Nkp46, Muc1/CD16a/Nkp46, or IL-16/CD16a/Nkp46, or the like.

In a preferred embodiment, the multispecific antibody is a trispecific antibody, comprising a first antigen-binding moiety that specifically binds to CD16a, a second antigen-binding moiety that specifically binds to Nkp46, and a third antigen-binding moiety that specifically binds to FOLR1;
the second antigen-binding moiety has the CDR1 as set forth in SEQ ID NO: 63, the CDR2 as set forth in SEQ ID NO: 65, and the CDR3 as set forth in SEQ ID NO: 70;
the third antigen-binding moiety has the CDR1 as set forth in SEQ ID NO: 58, the CDR2 as set forth in SEQ ID NO: 59, and the CDR3 as set forth in SEQ ID NO: 60;
preferably, the second antigen-binding moiety that specifically binds to Nkp46 and the third antigen-binding moiety that specifically binds to FOLR1 are VHHs;
preferably, the trispecific antibody is not fused to an Fc.

In one embodiment, the amino acid sequences of the aforementioned trispecific antibody are set forth in SEQ ID NOs: 47-51, respectively.

A sixth aspect of the present invention is to provide a polynucleotide molecule encoding the aforementioned anti-CD16a single-domain antibody, or the aforementioned Fc-fusion antibody, or the aforementioned humanized antibody, whose nucleotide sequences are set forth in SEQ ID NOs: 6-10, respectively, or wherein the amino acid sequence encoded by the nucleotide sequence is identical to the amino acid sequence encoded by any one of SEQ ID NOs: 6-10, or the nucleotide sequence has at least 95% sequence homology with any one of SEQ ID NOs: 6-10.

In one embodiment, the nucleic acid molecule encoding the anti-CD16a single-domain antibody has at least 90%, 91%, 92%, 93%, 94%, 95%, 96%, 97%, 98%, 99% or 100% sequence homology with a nucleotide sequence selected from the group consisting of SEQ ID NOs: 6-10, and the anti-CD16a single-domain antibody encoded thereby is capable of specifically binding to the CD16a protein.

A seventh aspect of the present invention is to provide a polynucleotide molecule encoding the aforementioned bispecific or multispecific antibody, whose nucleotide sequences are set forth in SEQ ID NOs: 52-56, respectively, or wherein the amino acid sequence encoded by the nucleotide sequence is identical to the amino acid sequence encoded by any one of SEQ ID NOs: 52-56, or the nucleotide sequence has at least 95% sequence homology with any one of SEQ ID NOs: 52-56.

In one embodiment, the polynucleotide molecule encoding the aforementioned bispecific or multispecific antibody has at least 90%, 91%, 92%, 93%, 94%, 95%, 96%, 97%, 98%, 99%, or 100% sequence homology with a nucleotide sequence selected from the group consisting of SEQ ID NOs: 52-56, and the bispecific or multispecific antibody encoded thereby is capable of specifically binding to the CD16a, FOLR1, or Nkp46 protein.

An eighth aspect of the present invention is to provide an expression vector, comprising the aforementioned polynucleotide molecule encoding the anti-CD16a single-domain antibody, or Fc-fusion antibody, or humanized antibody, or comprising the aforementioned polynucleotide molecule encoding the bispecific or multispecific antibody.

In a preferred embodiment, the expression vector used herein may be RJK-V4-hFC (in which a polynucleotide molecule encoding the anti-CD16a single-domain antibody, or the Fc-fusion antibody or the humanized antibody thereof is integrated into RJK-V4-hFC by means of genetic engineering), alternatively, other universal expression vectors may also be selected as needed.

A ninth aspect of the present invention is to provide a host cell capable of expressing the aforementioned anti-CD16a single-domain antibody, Fc-fusion antibody, humanized antibody, bispecific antibody, or multispecific antibody, or a host cell comprising the aforementioned expression vector. Preferably, the host cell is a bacterial cell, a fungal cell, or a mammalian cell.

In another preferred embodiment, the host cell includes a prokaryotic cell or a eukaryotic cell, including a bacterium or a fungus.

In a further preferred embodiment, the host cell is selected from the group consisting of an *Escherichia coli*, a yeast cell, a mammalian cell, a bacteriophage and a combination thereof.

In another preferred embodiment, the prokaryotic cell is selected from the group consisting of an *Escherichia coli*, a *Bacillus subtilis*, a *Lactobacillus*, a *Streptomyces*, a *Proteus mirabilis*, and a combination thereof.

In another preferred embodiment, the eukaryotic cell is selected from the group consisting of a *Pichia pastoris*, a *Saccharomyces cerevisiae*, a *Schizosaccharomyces* yeast, a *Trichoderma* fungus, and a combination thereof.

In another preferred embodiment, the eukaryotic cell is selected from the group consisting of an insect cell such as a *Spodoptera frugiperda* cell, a plant cell such as a *Nicotiana tabacum* cell, a BHK cell, a CHO cell, a COS cell, a myeloma cell, and a combination thereof.

In a further preferred embodiment, the host cell is an ExpiCHO-S cell in suspension.

In a further preferred embodiment, the host cell is a 293F cell in suspension.

A tenth aspect of the present invention is to provide a recombinant protein comprising the aforementioned anti-CD16a single-domain antibody. The recombinant protein may be the single-domain antibody as shown in any one of the aforementioned SEQ ID NOs: 1-5, or a single-domain antibody with at least 80% homology to any one of SEQ ID NOs: 1-5, or it can also be a multi-epitope binding antibody, a bispecific antibody, a multispecific antibody, or a multivalent antibody; for example, the multi-epitope binding antibody may be composed of more than one sequence selected from SEQ ID NOs: 1-5; the multivalent antibody may be composed of any one of the sequences of SEQ ID NOs: 1-5 arranged repeatedly multiple times; the multispecific antibody includes, but is not limited to, a trispecific antibody, a tetraspecific antibody; in addition, the recombinant protein may be a fragment, derivative, or analog of the aforementioned antibodies.

An eleventh aspect of the present invention is to provide a pharmaceutical composition comprising the aforementioned single-domain antibody that binds to CD16a or the aforementioned bispecific or multispecific antibody, and a pharmaceutically acceptable carrier. Typically, these substances can be formulated in a non-toxic, inert, and pharmaceutically acceptable aqueous carrier medium, where the pH is generally determined based on the isoelectric point of the antibody (the pH of the aqueous carrier medium should deviate from the isoelectric point of the antibody, differing by approximately 2 units). The formulated pharmaceutical composition may be administered via conventional routes, including (but not limited to): intravenous, transdermal administration (directly applying or patching a medicated plaster onto the affected area).

The pharmaceutical composition of the present invention contains the aforementioned single-domain antibody in a safe and effective amount (e.g., 0.001-99 wt%, preferably 0.01-90 wt%, more preferably 0.1-80 wt%) and a pharmaceutically acceptable carrier or excipient. Such carriers include (but are not limited to): saline, buffer, glucose, water, glycerol, ethanol, and a combination thereof. A pharmaceutical preparation should be adapted to the mode of administration. The pharmaceutical composition of the present invention can be formulated into an injectable form, for example, prepared by a conventional method using a physiological saline or an aqueous solution containing glucose and other adjuvants. Pharmaceutical compositions such as injections and solutions are preferably manufactured under sterile conditions.

A twelfth aspect of the present invention is to provide a medicament for use in treating a disease, comprising the aforementioned single-domain antibody for binding to the CD16a protein, or the aforementioned bispecific or multispecific antibody, as an active ingredient.

A thirteenth aspect of the present invention is to provide a kit for detecting the level of CD16a, comprising the aforementioned anti-CD16a single-domain antibody. In a preferred example of the invention, the kit further comprises a container, an instruction for use, a buffer, and the like.

In a preferred embodiment, the kit comprises an antibody that recognizes the CD16a protein, a lysis medium for lysing samples, and universal reagents and buffers required for detection, such as various buffers, detection labels, and detection substrates. The detection kit may be an *in vitro* diagnostic device.

In a preferred embodiment, the kit further comprises a secondary antibody and an enzyme, a fluorescent, or radioactive label for detection, as well as a buffer.

In a preferred embodiment, the secondary antibody of the kit may be an antibody against the aforementioned anti-CD16a single-domain antibody (acting as an anti-antibody), which may be a single-domain antibody, a monoclonal antibody, a polyclonal antibody, or any other form of antibody.

A thirteenth aspect of the present invention provides a method for producing an anti-CD16a single-domain antibody, comprising the steps of:
(a) culturing the host cell according to the ninth aspect of the present invention under a condition suitable for producing a single-domain antibody, thereby obtaining a culture containing the anti-CD16a single-domain antibody; and
(b) isolating or recovering the anti-CD16a single-domain antibody from the culture; and
(c) optionally, purifying and/or modifying the anti-CD16a single-domain antibody obtained in step (b).

A fourteenth aspect of the present invention is to provide use of the aforementioned anti-CD16a single-domain antibody, the aforementioned bispecific or multispecific antibody, or the aforementioned pharmaceutical composition in the manufacture of a medicament for treating a disease.

In a preferred embodiment, the disease refers to NK cell-mediated various disorders associated with CD16a.

In a preferred embodiment, the NK cell-mediated various disorders associated with CD16a include, but are not limited to, rheumatoid arthritis (RA), bone erosion, intraperitoneal abscess, inflammatory bowel disease, allotransplantation rejection, psoriasis, angiogenesis, atherosclerosis, asthma, multiple sclerosis, systemic lupus erythematosus (SLE), ocular surface disorders (e.g., dry eye disease), ankylosing spondylitis, psoriatic arthritis, and cancers (e.g., multiple myeloma and breast cancer).

In a preferred embodiment, the disease includes, but is not limited to, tumors, autoimmune diseases, metabolism-related diseases, and infectious diseases.

In a preferred embodiment, the infectious diseases include acute and chronic infectious diseases (e.g., bacterial infection or viral infection).

In a preferred embodiment, the tumors include solid tumors and hematological tumors.

In a preferred embodiment, the tumors include, but are not limited to, tumors of epithelial origin (adenomas and various types of carcinomas, including adenocarcinomas, squamous cell carcinomas, transitional cell carcinomas and other carcinomas); bladder cancer and urinary tract cancer; breast cancer; gastrointestinal cancer (including esophageal cancer, stomach (gastric) cancer, small intestine cancer, colon cancer, rectal cancer and anal cancer); liver cancer (hepatocellular carcinoma); cancers related to gallbladder and biliary tract system, exocrine pancreas and kidney; lung cancers (e.g., adenocarcinoma, small cell lung cancer, non-small cell lung cancer, bronchioloalveolar carcinoma, and mesothelioma); head and neck cancers (e.g., tongue cancer, oral cancer, laryngeal cancer, pharyngeal cancer, nasopharyngeal cancer, tonsil cancer, salivary gland cancer, nasal cancer and paranasal sinus cancer); cancers related to ovary, fallopian tube, peritoneum, vagina, vulva, penis, cervix, myometrium and endometrium; thyroid cancer (e.g., follicular thyroid carcinoma); adrenal cancer, prostate cancer, cancers related to skin and its appendages (e.g., melanoma, basal cell carcinoma, squamous cell carcinoma, keratoacanthoma, and dysplastic nevus); hematological malignancies (i.e., leukemias, lymphomas) and premalignant hematologic disorders and borderline malignancies, including hematological malignant tumors and lymphoid lineage related disorders (e.g., acute lymphocytic leukemia (ALL), chronic lymphocytic leukemia (CLL), B-cell lymphomas such as diffuse large B-cell lymphoma (DLBCL), follicular lymphoma, Burkitt's lymphoma, mantle cell lymphoma, T-cell lymphoma and leukemia, natural killer (NK) cell lymphoma, Hodgkin lymphoma, hairy cell leukemia, monoclonal gammopathy of undetermined significance, plasmacytoma, multiple myeloma and post-transplant lymphoproliferative disorders) as well as hematological malignant tumors and myeloid-related diseases (e.g., acute myeloid leukemia (AML), chronic myeloid leukemia (CML), chronic myelomonocytic leukemia (CMML), hypereosinophilic syndrome, myeloproliferative diseases such as polycythemia vera, essential thrombocythemia and primary myelofibrosis, myeloproliferative syndrome, myelodysplastic syndrome, and promyelocytic leukemia); tumors of mesenchymal origin, such as sarcomas of soft tissue, bone, or cartilage, for example, osteosarcoma, fibrosarcoma, chondrosarcoma, rhabdomyosarcoma, leiomyosarcoma, liposarcoma, angiosarcoma, Kaposi's sarcoma, Ewing's sarcoma, synovial sarcoma, epithelioid sarcoma, gastrointestinal stromal tumor, benign and malignant histiocytomas and dermatofibrosarcoma protuberans; tumors of the central or peripheral nervous system (e.g., astrocytoma, glioma and glioblastoma, meningioma, ependymoma, pinealoma, and schwannoma); endocrine tumors (e.g., pituitary tumor, adrenal tumor, islet cell tumor, parathyroid tumor, carcinoid tumor and medullary thyroid carcinoma); tumors of the eye and its appendages (e.g., retinoblastoma); germ cell and trophoblastic tumors (e.g., teratoma, seminoma, dysgerminoma, hydatidiform mole, and choriocarcinoma); pediatric and embryonic tumors (e.g., medulloblastoma, neuroblastoma, nephroblastoma, and primitive neuroectodermal tumor); or congenital or other syndromes that predispose a patient to malignant tumors (e.g., xeroderma pigmentosum).

In a preferred embodiment, the disease includes, but is not limited to, peripheral T-cell lymphoma, Hodgkin lymphoma, mycosis fungoides, anaplastic large cell lymphoma, pancreatic cancer, gastroesophageal junction cancer, gastric cancer, hepatocellular carcinoma, clear cell renal cell carcinoma, biliary tract cancer, colorectal cancer, non-small cell lung cancer, head and neck squamous cell carcinoma, diffuse large B-cell lymphoma, mantle cell lymphoma, marginal zone lymphoma, follicular lymphoma, multiple myeloma, acute myeloid leukemia, myelodysplastic syndrome, non-Hodgkin lymphoma, B-cell leukemia, and viral infection.

### Advantages of the invention

Compared with the prior art, the present invention has the following advantages:
(1) the single-domain antibody of the present invention specifically targets the CD16a protein with a proper spatial structure.
(2) the single-domain antibody obtained in this invention has a flexible expression system choice and can be expressed in either a prokaryotic system or a eukaryotic system such as yeast cells or mammalian cells, moreover, its expression in the prokaryotic expression system has a low cost, which can reduce subsequent production expenses.
(3) the single-domain antibody obtained in this invention is easy to be modified into multiple combination forms of the antibody, and multivalent or multi-specific antibodies can be obtained through the simple tandem connection in a genetic engineering manner, moreover, it has a very low immune heterogeneity and will not trigger a strong immune response even without humanization modification.
(4) the single-domain antibody obtained in this invention has a wider affinity range, which can vary from the nM level to the pM level before affinity maturation, offering diverse options for antibodies intended for subsequent diverse purposes;
(5) the single-domain antibody against CD16a obtained in this invention possesses a high antigen-binding ability and specificity, and exhibits an excellent redirected killing capability, which can effectively mediate the ADCC effect. The single-domain antibody can be combined with other antigen-binding moieties to form a bispecific or multispecific antibody, or can be used as a part of a chimeric antigen receptor (CAR), or can be assembled into any other form of antibody.

The trispecific antibody FOLR1/CD16a/NKp46 prepared on the basis of the anti-CD16a single-domain antibody (wherein FOLR1/CD16a/NKp46 is just one example, and CD16a can also be combined with other tumor surface antigen targets) still exhibits an unexpectedly superior ADCC effect even in the absence of an immunoglobulin Fc.

### DESCRIPTION OF DRAWINGS

In order to more clearly illustrate the technical solutions of this application, the accompanying drawings, which are to be used in the description of the examples, will be briefly introduced below, and it is apparent that, for a person of ordinary skill in the art, other drawings can be obtained based on these drawings without creative efforts.
Figure 1 shows the library enrichment for screening antibodies targeting CD16a in Example 3, with the left panel showing the library enrichment for screening antibodies targeting CD16a-V176, and the right panel showing the library enrichment for screening antibodies targeting CD16a-F176;
Figure 2 shows the binding dose-effect curves of the CD16a-hFc antibody to human CD16a (V176) in Example 12 (14A3);
Figure 3 shows the binding dose-effect curves of the CD16a-hFc antibody to human CD16a (V176) in Example 12 (23D1);
Figure 4 shows the binding dose-effect curves of the CD16a-hFc antibody to human CD16a (V176) in Example 12 (15E2, 13G6);
Figure 5 shows the binding dose-effect curves of the CD16a-hFc antibody to human CD16a (V176) in Example 12 (4B5);
Figure 6 shows the binding dose-effect curves of the CD16a-hFc antibody to human CD16a (F176) in Example 12 (4B5);
Figure 7 shows the binding dose-effect curves of the CD16a-hFc antibody to human CD16a (F176) in Example 12 (15E2, 13G6);
Figure 8 shows the binding dose-effect curves of the CD16a-hFc antibody to human CD16a (F176) in Example 12 (14A3);
Figure 9 shows the binding dose-effect curves of the CD16a-hFc antibody to human CD16a (F176) in Example 12 (23D1);
Figure 10 shows the RKA assay results for the CD16a-hFc antibody (4B5);
Figure 11 shows the RKA assay results for the CD16a-hFc antibody (15E2);
Figure 12 shows the RKA assay results for the CD16a-hFc antibody (13G6, 14A3);
Figure 13 shows the RKA assay results for the CD16a-hFc antibody (23D1);
Figure 14 shows the ADCC assay results for the CD16a-hFc (4B5);
Figure 15 shows the ADCC assay results for the CD16a-hFc (15E2);
Figure 16 shows the ADCC assay results for the CD16a-hFc (13G6, 14A3);
Figure 17 shows the ADCC assay results for the CD16a-hFc (23D1);
Figure 18 shows the structure of the FOLR1/Nkp46/CD16a trispecific antibody;
Figures 19-21 show the ADCC assay results for the FOLR1/Nkp46/CD16a trispecific antibody;
Figure 22 shows the ADCC assay results for the FOLR1/Nkp46 bispecific antibody.

### Detailed Description of The Invention

The present disclosure will be further described in detail below with reference to examples, so as to enable a person skilled in the art to carry out the disclosure based on the text of this specification.

As used herein, "single-domain antibody" (sdAb, also referred to as nanobody or VHH by the developer Ablynx) is well known to those skilled in the art. A single-domain antibody is an antibody whose complementarity-determining region is part of a single-domain polypeptide. Thus, a single-domain antibody comprises a single complementarity-determining region (a single CDR1, a single CDR2, and a single CDR3). Examples of the single-domain antibody are a heavy chain-only antibody (which naturally contains no light chains), a single-domain antibody derived from a conventional antibody, and an engineered antibody.

The single-domain antibody may be derived from any species, including mouse, human, camel, llama, goat, rabbit and cattle. For example, naturally occurring VHH molecules may be derived from antibodies provided by Camelidae species (such as camel, dromedary, llama and guanaco). Like an intact antibody, the single-domain antibody can selectively bind to a specific antigen. The single-domain antibody may contain only a variable domain of an immunoglobulin chain, with the domain having CDR1, CDR2, and CDR3 as well as framework regions.

As used herein, the term "sequence homology" refers to the degree to which two (nucleotide or amino acid) sequences in an alignment have the same residues at the same positions, and is usually expressed as a percentage. Preferably, homology is determined over the entire length of the sequences being compared. Therefore, two copies with identical sequences share 100% homology.

As used herein, the term "Fc-fusion antibody" refers to a novel protein produced by fusing the Fc segment of an antibody with a functional protein molecule having a certain biological activity by genetic engineering technologies.

The term "humanized antibody" refers to an antibody obtained by fusing the heavy chain variable region of an antibody of interest (such as an animal antibody) with the constant region of a human antibody, or an antibody obtained by transplanting the complementarity-determining region (CDR1-3 sequences) of an antibody of interest into the variable region of a human antibody, or an antibody obtained by mutating amino acids of an antibody of interest based on features of the framework region (FR1-4) of a human antibody. The humanized antibody may be obtained by a synthetic method or a site-directed mutagenesis method.

In the present disclosure, the single-domain antibody against CD16a may also be obtained from sequences having high homology with the sequences of CDR1-3 disclosed in the present invention. In some embodiments, sequences having "at least 80% homology", or "at least 85% homology", "at least 90% homology", "at least 95% homology", or "at least 98% homology" to those in SEQ ID NOs: 1-5 can all achieve the purpose of the present disclosure.

In some embodiments, the purpose of the invention can also be achieved by sequences with only one or a few amino acids substituted, such as those containing 1, 2, 3, 4, 5, 6, 7, 8, 9, or 10 conservative amino acid substitutions, with respect to the sequences in SEQ ID NOs: 1-5. In fact, when determining the degree of sequence homology between two amino acid sequences or when identifying the combination of CDR1, CDR2 and CDR3 in a single-domain antibody, one skilled in the art may take into account the so-called "conservative" amino acid substitution, and in the case of substitution, the substitution will preferably be a conservative amino acid substitution, where the conservative amino acid substitution is generally described as an amino acid substitution in which an amino acid residue is replaced by another amino acid residue with a similar chemical structure, and this substitution has little or substantially no effect on the function, activity or other biological properties of the polypeptide. The conservative amino acid substitutions are common in the art, for example, a conservative amino acid substitution means the substitution of one or a few amino acids within the following groups (a)-(d) by another one or few amino acids within the same group: (a) polar negatively-charged residues and uncharged amides thereof: Asp, Asn, Glu, and Gln; (b) polar positively-charged residues: His, Arg, and Lys; (c) aromatic residues: Phe, Trp, and Tyr; and (d) aliphatic nonpolar or weakly-polar residues: Ala, Ser, Thr, Gly, Pro, Met, Leu, Ile, Val, and Cys. The following are particularly preferred conservative amino acid substitutions: Asp is substituted by Glu; Asn is substituted by Gln or His; Glu is substituted by Asp; Gln is substituted by Asn; His is substituted by Asn or Gln; Arg is substituted by Lys; Lys is substituted by Arg or Gln; Phe is substituted by Met, Leu, or Tyr; Trp is substituted by Tyr; Tyr is substituted by Phe or Trp; Ala is substituted by Gly or Ser; Ser is substituted by Thr; Thr is substituted by Ser; Gly is substituted by Ala or Pro; Met is substituted by Leu, Tyr, or Ile; Leu is substituted by Ile or Val; Ile is substituted by Leu or Val; Val is substituted by Ile or Leu; or Cys is substituted by Ser. Furthermore, those skilled in the art will be aware that the inventiveness of a single-domain antibody is embodied in the CDR1-3 regions, while the framework region sequences FR1-4 are not unchangeable, and the sequences of FR1-4 can be conservative sequence variants of the sequences disclosed in the present disclosure.

The preferred host cell in the present invention is a bacterial cell, a fungal cell, or a mammalian cell.

In this application, a protein of interest and the truncated form thereof are prepared by genetic engineering technologies, and then the obtained antigen protein is used to immunize Bactrian camels from Alxa in Inner Mongolia, after multiple immunizations, peripheral blood lymphocytes or spleen cells of the camels are obtained, subsequently, the coding sequences of the camelid antibody variable regions are recombined into a phage display vector by means of genetic engineering, and specific antibodies against the antigen protein are screened out using the phage display technology, furthermore, their ability to bind to the antigen is detected, along with their applications, including the application in the treatment of autoimmune diseases.

Now, the above technical solutions will be split apart and interpreted in detail through specific examples:

### Example 1: Preparation of recombinant extracellular domain protein of human CD16a

The human recombinant extracellular domain protein used in the application was obtained by in-house expression and purification, and the specific design scheme of the expression vector for the human recombinant CD16a protein was as follows:
(1) the coding sequence for CD16a was retrieved from NCBI with the accession number NM_000569.7, and the corresponding amino acid sequence generated by this sequence had the accession number NP_000560.6.
(2) the nucleotide sequences encoding amino acids 17-208 of CD16a (CD16a-V176, CD16a-F176) were cloned into the vector pcDNA3.4 by gene synthesis. The constructed vector was subjected to Sanger sequencing and aligned against the original sequence, after confirmation, the recombinant plasmid was extracted in large quantities, and endotoxins were removed, followed by transfection into 293F cells in suspension for expression and purification of the protein of interest, as a result, the purity reached 90% or higher, meeting the requirements for animal immunization.

There were two types of antigens, namely, the first one comprises amino acids 17-208 of CD16a with the amino acid at position 176 being F, referred to as CD16a-F176; the second one comprises amino acids 17-208 of CD16a with the amino acid at position 176 being V, referred to as CD16a-V176. Both antigens were separately prepared and purified.

### Example 2: Construction of single-domain antibody library against CD16a protein

1 mg of the human recombinant CD16a proteins (CD16a-F176 and CD16a-V176) obtained by purification from Example 1 were mixed with an equal volume of Freund's complete adjuvant, and the resultant mixture was used to immunize Bactrian camels from Alxa in Inner Mongolia, respectively. The immunization was conducted once a week for a total of 7 consecutive times, specifically, except for the initial immunization, the remaining six immunizations were performed by mixing 1 mg of the CD16a protein with an equal volume of Freund's incomplete adjuvant for animal immunization, and this immunization process was intended to intensified stimulation of the camels to induce the generation of antibodies against the CD16a protein. CD16a-V176 and CD16a-F176 were not mixed, and the animals were separately immunized to obtain antibodies.

After the animal immunization was completed, 150 mL of peripheral blood lymphocytes were drawn from the camels, and the RNA was extracted from the cells. The extracted total RNA was used to synthesize cDNA, and the VHH (antibody heavy chain variable region) was amplified using the cDNA as a template through a nested PCR reaction.

Next, the pMECS vector and VHH fragment were digested with restriction endonucleases, respectively, and the cleaved fragments were then ligated to the vector. The ligated fragments were electroporated into competent TG1 cells to construct a phage display library for the CD16a protein and the library capacity was measured. The resulting library capacity is approximately 1×10⁹ in size. Meanwhile, the correct insertion rate of the fragment of interest in the library was detected by colony PCR identification.

The results showed that, after PCR amplification of 30 bacterial colonies randomly selected from the library, 29 clones could be amplified to produce bands with the expected size, while 1 clone was amplified with incorrect bands, so the correct insertion rate was 29÷30×100%≈96.7%.

### Example 3: Screening single-domain antibodies against CD16a protein

200 µL of recombinant TG1 cells in Example 2 were cultured in a 2×TY medium, during which 40 µL of VCSM13 helper phages were added to infect the TG1 cells, followed by culturing overnight to amplify the phages, which were precipitated using PEG/NaCl on the next day, and the amplified phages were collected by centrifugation.

500 µg of the CD16a protein diluted in 100 mM NaHCO₃ at pH 8.3 was coupled onto an ELISA plate and left to stand at 4°C overnight, and negative control wells (culture medium control) were also set up; on the next day, 200 µL of 3% skimmed milk was added, followed by blocking at room temperature for 2 hours; after blocking, 100 µl of amplified phage library (approximately 2×10¹¹ phage particles) was added to react at room temperature for 1 hour; after 1 hour of reaction, the plate was washed with PBS+0.05% Tween-20 for 15 times to wash away unbound phages.

The phages specifically bound to the CD16a protein were dissociated using trypsin at a final concentration of 25 mg/mL and then used to infect *Escherichia coli* TG1 cells in a logarithmic growth phase, after incubation at 37°C for 1 hour, the resulting phages were collected for the next round of screening, with the same screening process repeated for another round, gradually achieving enrichment.

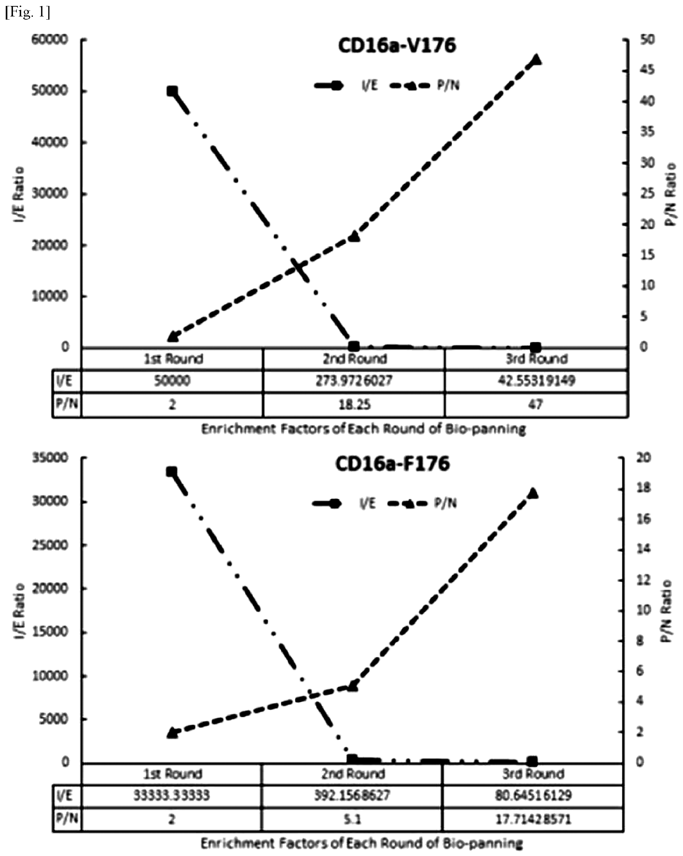

When an enrichment factor reached 10 or higher, the enrichment effect was as shown in Figure 1.

In Figure 1, P/N = the number of monoclonal bacteria grown after TG1 bacteria were infected with phages eluted from positive wells / the number of monoclonal bacteria grown after TG1 bacteria were infected with phages eluted from negative wells in the bio-panning, and this parameter would gradually increase following enrichment; I/E = the total amount of phages added to positive wells in each round of bio-panning / the total amount of phages eluted from positive wells in each round of bio-panning, and this parameter would gradually approach 1 following enrichment.

### Example 4: Screening positive clones specifically against CD16a by phage enzyme-linked immunosorbent assay (ELISA)

Three rounds of screening for single-domain antibodies against the CD16a protein were conducted according to the screening method in the above Example 3, until a enrichment factor of phages against the CD16a protein reached 10 or higher. After the screening, 384 single colonies were picked from screened positive clones and inoculated into 96 deep-well plates containing a 2×TY medium supplemented with 100 µg/mL ampicillin, respectively, with a blank control set up as well, after culturing at 37°C to a logarithmic phase, IPTG was added to a final concentration of 1 mM, and allowed to incubate at 28°C overnight.

Crude antibodies were obtained by the osmotic burst method; recombinant CD16a proteins were diluted in 100 mM NaHCO₃ at pH 8.3, respectively, and microtiter plates (ELISA plates) were then coated with 100 µg of the proteins and left at 4°C overnight. 100 µL of the obtained crude antibody extract was transferred to the ELISA plates into which the antigen had been added, allowed to incubate at room temperature for 1 hour; unbound antibodies were washed away with PBST, and 100 µl of Mouse Anti-HA tag Antibody (HRP) (mouse anti-HA horseradish peroxidase-labeled antibody, Thermo Fisher) at 1:2000 dilution was added and incubated at room temperature for 1 hour; the unbound antibodies were washed away with PBST, and a horseradish peroxidase chromogenic solution was added, after 15 minutes of reaction at 37°C, a stop solution was added, and the absorbance value was read at a wavelength of 450 nm using a microplate reader.

When the OD value of a sample well was more than 5 times that of the control well, the sample well was determined to be a positive clone well; the bacteria in the positive clone wells were transferred into an LB medium containing 100 µg/mL ampicillin for plasmid extraction and sequencing.

Gene sequences of each clone strain were analyzed according to the sequence alignment software VectorNTI, wherein strains with identical sequences of CDR1, CDR2, and CDR3 were identified as the same clone, while strains with different sequences were identified as different clone, and ultimately, single-domain antibodies specifically targeting the CD16a protein were yielded.

The amino acid sequences of the antibodies followed the FR1-CDR1-FR2-CDR2-FR3-CDR3-FR4 structure, constituting the entire VHH. The obtained recombinant plasmids for the single-domain antibodies can be expressed in a prokaryotic system to ultimately obtain single-domain antibody proteins.

Among them, the single-domain antibody clones screened against the antigen CD16a-F176 include 15E2 and 4B5; the single-domain antibody clones screened against the antigen CD16a-V176 include 14A3, 23D1, and 13G6.

Other single-domain antibody clones (sequences of which were not shown) were also screened by using the antigens CD16a-F176 and CD16a-V176: 18E4, 10B6, 8G8, 15A7, and 24C6W.

CDR and FR sequences of the five single-domain antibodies were shown in Tables 1-7, and amino acid sequences and nucleotide sequences of the five single-domain antibodies were shown in Table 8 and Table 9, respectively.

**Table 1 CDR1 sequences of five single-domain antibodies**

| Actual clone ID | CDR1 | SEQ ID |
|---|---|---|
| 15E2 | GGTFRIYA | SEQ ID NO: 11 |
| 4B5 | | |
| 14A3 | GRTFSYLA | SEQ ID NO: 12 |
| 23D1 | | |
| 13G6 | GRTFRIYA | SEQ ID NO: 13 |

**Table 2 CDR2 sequences of five single-domain antibodies**

| **Actual clone ID** | **CDR2** | **SEQ ID** |
|---|---|---|
| 14A3 | ASWGGGST | SEQ ID NO: 14 |
| 13G6 | IRLMRGIT | SEQ ID NO: 15 |
| 15E2 | ISMMRGIT | SEQ ID NO: 16 |
| 4B5 | | |
| 23D1 | SSWGGGTS | SEQ ID NO: 17 |

**Table 3 CDR3 sequences of five single-domain antibodies**

| Actual clone ID | CDR3 | SEQ ID |
|---|---|---|
| 13G6 | AAVGSGGDFYTPSTSGEYRY | SEQ ID NO: 18 |
| 15E2 | AIRERPPPMGWGLAGISYDY | SEQ ID NO: 19 |
| 4B5 | AKAVDKSPYGGWGWGPYDD | SEQ ID NO: 20 |
| 14A3 | VVGRRFKGSVYYTPNEYEY | SEQ ID NO: 21 |
| 23D1 | | |

**Table 4 FR1 sequences of five single-domain antibodies**

| Actual clone ID | FR1 | SEQ ID |
|---|---|---|
| 15E2 | QVQLVESGGGLVQAGDSLRLSCAAS | SEQ ID NO: 22 |
| 4B5 | QVQLVESGGGLVQAGGSLRLSCAAS | SEQ ID NO: 23 |
| 23D1 | QVQLVESGGGLVQAGGSLRLSCAVS | SEQ ID NO: 24 |
| 13G6 | QVQLVESGGGLVQPGGSLRLSCAAS | SEQ ID NO: 25 |
| 14A3 | QVQLVESGGGLVQPGGSLRLSCEVS | SEQ ID NO: 26 |

**Table 5 FR2 sequences of five single-domain antibodies**

| Actual clone ID | FR2 | SEQ ID |
|---|---|---|
| 14A3 | LGWFRQAPGKEREFVAA | SEQ ID NO: 27 |
| 23D1 | MGWFRQAPGKEREFVAA | SEQ ID NO: 28 |
| 15E2 | MGWFRQAPGKEREFVGS | SEQ ID NO: 29 |
| 4B5 | | |
| 13G6 | | |

**Table 6 FR3 sequences of five single-domain antibodies**

| Actual clone ID | FR3 | SEQ ID |
|---|---|---|
| 13G6 | DYADSVKGRFTISRDNAKFTMYLQMNTLKPED TAVYYC | SEQ ID NO: 30 |
| 4B5 | DYAESVKGRFTISRDNAKFTMYLQMNSLKSED TAVYYC | SEQ ID NO: 31 |
| 15E2 | DYAESVKGRFTISRDNAKFTMYLQMNTLKPED TAVYYC | SEQ ID NO: 32 |
| 23D1 | GYADSVKGRFAISRDNAKNTQYLQMNSLKPED TAVYYC | SEQ ID NO: 33 |
| 14A3 | GYADSVKGRFAISRDNVKNTQYLQMNSLKPED TAVYYC | SEQ ID NO: 34 |

**Table 7 FR4 sequences of five single-domain antibodies**

| Actual clone ID | FR4 | SEQ ID |
|---|---|---|
| 15E2 | | |
| 4B5 | | |
| 13G6 | WGQGTQVTVSS | SEQ ID NO: 35 |
| 14A3 | | |
| 23D1 | | |

**Table 8 Amino acid sequences of five single-domain antibodies**

| **Actual clone ID** | **Amino acid sequence** | **SEQ ID** |
|---|---|---|
| 4B5 | QVQLVESGGGLVQAGGSLRLSCAASGGTFRIYAMGWFRQAPGKEREFVGSISMMRGIT DYAESVKGRFTISRDNAKFTMYLQMNSLKSEDTAVYYCAKAVDKSPYGGWGWGPYD DWGQGTQVTVSS | SEQ ID NO: 1 |
| 15E2 | QVQLVESGGGLVQAGDSLRLSCAASGGTFRIYAMGWFRQAPGKEREFVGSISMMRGIT DYAESVKGRFTISRDNAKFTMYLQMNTLKPEDTAVYYCAIRERPPPMGWGLAGISYD YWGQGTQVTVSS | SEQ ID NO: 2 |
| 13G6 | QVQLVESGGGLVQPGGSLRLSCAASGRTFRIYAMGWFRQAPGKEREFVGSIRLMRGIT DYADSVKGRFTISRDNAKFTMYLQMNTLKPEDTAVYYCAAVGSGGDFYTPSTSGEYR YWGQGTQVTVSS | SEQ ID NO: 3 |
| 14A3 | QVQLVESGGGLVQPGGSLRLSCEVSGRTFSYLALGWFRQAPGKEREFVAAASWGGGS TGYADSVKGRFAISRDNVKNTQYLQMNSLKPEDTAVYYCVVGRRFKGSVYYTPNEYE YWGQGTQVTVSS | SEQ ID NO: 4 |
| 23D1 | QVQLVESGGGLVQAGGSLRLSCAVSGRTFSYLAMGWFRQAPGKEREFVAASSWGGG TSGYADSVKGRFAISRDNAKNTQYLQMNSLKPEDTAVYYCVVGRRFKGSVYYTPNEY EYWGQGTQVTVSS | SEQ ID NO: 5 |

**Table 9 Nucleic acid sequences of five single-domain antibodies**

| **Actua l clone ID** | **Nucleic acid sequence** | **SEQ ID** |
|---|---|---|
| 4B5 | CAGGTGCAGCTGGTGGAGAGCGGCGGCGGCCTGGTGCAGGCCGGCGGCAGCCTGA GGCTGAGCTGCGCCGCCAGCGGCGGCACCTTCAGGATCTACGCCATGGGCTGGTTC AGGCAGGCCCCCGGCAAGGAGAGGGAGTTCGTGGGCAGCATCAGCATGATGAGGG GCATCACCGACTACGCCGAGAGCGTGAAGGGCAGGTTCACCATCAGCAGGGACAA CGCCAAGTTCACCATGTACCTGCAGATGAACAGCCTGAAGAGCGAGGACACCGCC GTGTACTACTGCGCCAAGGCCGTGGACAAGAGCCCCTACGGCGGCTGGGGCTGGG GCCCCTACGACGACTGGGGCCAGGGCACCCAGGTGACCGTGAGCAGC | SEQ ID NO: 6 |
| 15E2 | CAGGTGCAGCTGGTGGAGAGCGGCGGCGGCCTGGTGCAGGCCGGCGACAGCCTGA GGCTGAGCTGCGCCGCCAGCGGCGGCACCTTCAGGATCTACGCCATGGGCTGGTTC AGGCAGGCCCCCGGCAAGGAGAGGGAGTTCGTGGGCAGCATCAGCATGATGAGGG GCATCACCGACTACGCCGAGAGCGTGAAGGGCAGGTTCACCATCAGCAGGGACAA CGCCAAGTTCACCATGTACCTGCAGATGAACACCCTGAAGCCCGAGGACACCGCC GTGTACTACTGCGCCATCAGGGAGAGGCCCCCCCCCATGGGCTGGGGCCTGGCCG GCATCAGCTACGACTACTGGGGCCAGGGCACCCAGGTGACCGTGAGCAGC | SEQ ID NO: 7 |
| 13G6 | CAGGTGCAGCTGGTGGAGAGCGGCGGCGGCCTGGTGCAGCCCGGCGGCAGCCTGA GGCTGAGCTGCGCCGCCAGCGGCAGGACCTTCAGGATCTACGCCATGGGCTGGTTC AGGCAGGCCCCCGGCAAGGAGAGGGAGTTCGTGGGCAGCATCAGGCTGATGAGGG GCATCACCGACTACGCCGACAGCGTGAAGGGCAGGTTCACCATCAGCAGGGACAA CGCCAAGTTCACCATGTACCTGCAGATGAACACCCTGAAGCCCGAGGACACCGCC GTGTACTACTGCGCCGCCGTGGGCAGCGGCGGCGACTTCTACACCCCCAGCACCAG CGGCGAGTACAGGTACTGGGGCCAGGGCACCCAGGTGACCGTGAGCAGC | SEQ ID NO: 8 |
| 14A3 | CAGGTGCAGCTGGTGGAGAGCGGCGGCGGCCTGGTGCAGCCCGGCGGCAGCCTGA GGCTGAGCTGCGAGGTGAGCGGCAGGACCTTCAGCTACCTGGCCCTGGGCTGGTTC AGGCAGGCCCCCGGCAAGGAGAGGGAGTTCGTGGCCGCCGCCAGCTGGGGCGGCG GCAGCACCGGCTACGCCGACAGCGTGAAGGGCAGGTTCGCCATCAGCAGGGACAA CGTGAAGAACACCCAGTACCTGCAGATGAACAGCCTGAAGCCCGAGGACACCGCC GTGTACTACTGCGTGGTGGGCAGGAGGTTCAAGGGCAGCGTGTACTACACCCCCAA CGAGTACGAGTACTGGGGCCAGGGCACCCAGGTGACCGTGAGCAGC | SEQ ID NO: 9 |
| 23D1 | CAGGTGCAGCTGGTGGAGAGCGGCGGCGGCCTGGTGCAGGCCGGCGGCAGCCTGA GGCTGAGCTGCGCCGTGAGCGGCAGGACCTTCAGCTACCTGGCCATGGGCTGGTTC AGGCAGGCCCCCGGCAAGGAGAGGGAGTTCGTGGCCGCCAGCAGCTGGGGCGGCG GCACCAGCGGCTACGCCGACAGCGTGAAGGGCAGGTTCGCCATCAGCAGGGACAA CGCCAAGAACACCCAGTACCTGCAGATGAACAGCCTGAAGCCCGAGGACACCGCC GTGTACTACTGCGTGGTGGGCAGGAGGTTCAAGGGCAGCGTGTACTACACCCCCAA CGAGTACGAGTACTGGGGCCAGGGCACCCAGGTGACCGTGAGCAGC | SEQ ID NO: 10 |

### Example 5: Construction of humanized FOLR1/Nkp46 bispecific antibody domains

Single-domain antibodies against FOLR1 and Nkp46, namely the FOLR1-4F4 single-domain antibody and the Nkp46-7F10 single-domain antibody, were respectively obtained by screening with reference to the methods in Examples 1-4; the specific screening process differed with those in Examples 1-4 only in the antigen preparation procedure .

### Preparation of recombinant extracellular domain protein of human Nkp46:

The human recombinant extracellular domain protein used in the application was obtained by in-house expression and purification, and the specific design scheme of the expression vector for the human recombinant Nkp46 protein was as follows:
(1) the coding sequence for Nkp46 was retrieved from NCBI with the accession number BC064806.1, and the corresponding amino acid sequence generated by this sequence had the accession number AAH64806.1.
(2) the nucleotide sequence encoding amino acids 22-254 of Nkp46 was cloned into the vector pcDNA3.4 by gene synthesis. The constructed vector was subjected to Sanger sequencing and aligned against the original sequence, after confirmation, the recombinant plasmid was extracted in large quantities, and endotoxins were removed, followed by transfection into 293F cells in suspension for expression and purification of the protein of interest, as a result, the purity reached 90% or higher, meeting the requirements for animal immunization.

### Preparation of recombinant extracellular domain protein of human FOLR1:

The human recombinant extracellular domain protein used in the application was obtained by in-house expression and purification, and the specific design scheme of the expression vector for the human recombinant FOLR1 protein was as follows:
(1) the coding sequence for FOLR1 was retrieved from NCBI with the accession number NM_000802.3, and the corresponding amino acid sequence generated by this sequence had the accession number NP_000793.1.
(2) the nucleotide sequence encoding amino acids 25-233 of FOLR1 was cloned into the vector pcDNA3.4 by gene synthesis. The constructed vector was subjected to Sanger sequencing and aligned against the original sequence, after confirmation, the recombinant plasmid was extracted in large quantities, and endotoxins were removed, followed by transfection into 293F cells in suspension for expression and purification of the protein of interest, as a result, the purity reached 90% or higher, meeting the requirements for animal immunization.

Sequences of the screened FOLR1-4F4 single-domain antibody were shown in Table 10 and Table 11. Humanization modification was performed on the basis thereof, and the sequences of 4F4 modified by humanization were shown in Table 12, wherein the modified regions included FR1, CDR1, CDR2, CDR3, and FR4, as indicated specifically by the underlined parts in Table 12.

Sequences of the screened Nkp46-7F10 single-domain antibody were shown in Table 13 and Table 14. Humanization modification was performed on the basis thereof, and the sequences of 7F10 modified by humanization were shown in Table 15, wherein the modified regions included CDR3, as indicated specifically by the underlined parts in Table 15.

The humanized 4F4 and the humanized 7F10 were linked to form a humanized FOLR1/Nkp46, and the sequences were shown in Table 16, wherein the humanized 4F4 was shown as amino acids 1-125 of SEQ ID NO: 45, the linker between the humanized 4F4 and the humanized 7F10 was shown as amino acids 126-140 of SEQ ID NO: 45 (GGGGSGGGGSGGGGS), and the humanized 7F10 was shown as amino acids 141-267 of SEQ ID NO: 45.

**Table 10 CDR and FR sequences of FOLR1 (4F4)**

| **FR1** | **CDR1** | **FR2** | **CDR2** | **FR3** | **CDR3** | **FR4** |
|---|---|---|---|---|---|---|
| QVQLVESGG GSVQAGGSLR LSCAAS | DGTYR RYC | MGWFRQAP GKEREKVA A | IYTGDG TT | YYADSVKGRFTISQEN AKNTMYLQMNSLKPE DTAMYYC | AADRSGC SVAWSYS SFED | WGQGTQ VTVSS |
| SEQ ID NO: 36 | SEQ ID NO: 37 | SEQ ID NO: 38 | SEQ ID NO: 39 | SEQ ID NO: 40 | SEQ ID NO: 41 | SEQ ID NO: 42 |

**Table 11 Amino acid and nucleic acid sequences of FOLR1 (4F4)**

| | Sequence | SEQ ID |
|---|---|---|
| Amin o acid seque nce | QVQLVESGGGSVQAGGSLRLSCAASDGTYRRYCMGWFRQAPGKEREKVAAIYTGDGTTY YADSVKGRFTISQENAKNTMYLQMNSLKPEDTAMYYCAADRSGCSVAWSYSSFEDWGQG TQVTVSS | SEQ ID NO: 43 |
| Nucle ic acid seque nce | CAGGTGCAGCTGGTGGAGAGCGGCGGCGGCAGCGTGCAGGCCGGCGGCAGCCTGAGGC TGAGCTGCGCCGCCAGCGACGGCACCTACAGGAGGTACTGCATGGGCTGGTTCAGGCA GGCCCCCGGCAAGGAGAGGGAGAAGGTGGCCGCCATCTACACCGGCGACGGCACCACC TACTACGCCGACAGCGTGAAGGGCAGGTTCACCATCAGCCAGGAGAACGCCAAGAACA CCATGTACCTGCAGATGAACAGCCTGAAGCCCGAGGACACCGCCATGTACTACTGCGCC GCCGACAGGAGCGGCTGCAGCGTGGCCTGGAGCTACAGCAGCTTCGAGGACTGGGGCC AGGGCACCCAGGTGACCGTGAGCAGC | SEQ ID NO: 44 |

**Table 12 CDR and FR sequences of FOLR1 (humanized 4F4)**

| **FR1** | **CDR1** | **FR2** | **CDR2** | **FR3** | **CDR3** | **FR4** |
|---|---|---|---|---|---|---|
| QVQLVESGGG LVKPGGSLRL SCAAS | DATYR RYC | MGWFRQAP GKEREKVA A | IYTGDA TT | YYADSVKGRFTISQEN AKNTMYLQMNSLKPE DTAMYYC | AADRSGCSV AFSYSSFED | WGQGT LVTVSS |
| SEQ ID NO: 57 | SEQ ID NO: 58 | SEQ ID NO: 38 | SEQ ID NO: 59 | SEQ ID NO: 40 | SEQ ID NO: 60 | SEQ ID NO: 61 |

**Table 13 CDR and FR sequences of NkP46 (7F10)**

| **FR1** | **CDR1** | **FR2** | **CDR2** | **FR3** | **CDR3** | **FR4** |
|---|---|---|---|---|---|---|
| QVQLVESGGG LVQAGGSLRL SCAAS | GRTFS SYA | MGWFRQAP GKEREFVA G | ISWSGDS T | YYADSVKGRFTISRDN AKNTVGLQMNSLKPE DTAVYYC | AAASGQVQ TVVVVTDR WYHY | WGQGT QVTVSS |
| SEQ ID NO: 62 | SEQ ID NO: 63 | SEQ ID NO: 64 | SEQ ID NO: 65 | SEQ ID NO: 66 | SEQ ID NO: 67 | SEQ ID NO: 68 |

**Table 14 Amino acid sequence of NkP46 (7F10)**

| | Sequence | SEQ ID |
|---|---|---|
| Amin o acid seque nce | QVQLVESGGGLVQAGGSLRLSCAASGRTFSSYAMGWFRQAPGKEREFVAGISWSGDSTYYA DSVKGRFTISRDNAKNTVGLQMNSLKPEDTAVYYCAAASGQVQTVVVVTDRWYHYWGQG TQVTVSS | SEQ ID NO: 69 |

**Table 15 CDR and FR sequences of NkP46 (humanized 7F10)**

| **FR1** | **CDR1** | **FR2** | **CDR2** | **FR3** | **CDR3** | **FR4** |
|---|---|---|---|---|---|---|
| QVQLVESGGG LVQAGGSLRL SCAAS | GRTFS SYA | MGWFRQAP GKEREFVA G | ISWSGDS T | YYADSVKGRFTISRDN AKNTVGLQMNSLKPE DTAVYYC | AAASGQVQ TVVVVTDR FYHY | WGQGT QVTVSS |
| SEQ ID NO: 62 | SEQ ID NO: 63 | SEQ ID NO: 64 | SEQ ID NO: 65 | SEQ ID NO: 66 | SEQ ID NO: 70 | SEQ ID NO: 68 |

**Table 16 Amino acid and nucleic acid sequences of humanized FOLR1/Nkp46 bispecific antibody**

| | Sequence | SEQ ID |
|---|---|---|
| Amino acid seque nce | QVQLVESGGGLVKPGGSLRLSCAASDATYRRYCMGWFRQAPGKEREKVAAIYTGDATTYYA DSVKGRFTISQENAKNTMYLQMNSLKPEDTAMYYCAADRSGCSVAFSYSSFEDWGQGTLVT VSSGGGGSGGGGSGGGGSQVQLVESGGGLVQAGGSLRLSCAASGRTFSSYAMGWFRQAPGK EREFVAGISWSGDSTYYADSVKGRFTISRDNAKNTVGLQMNSLKPEDTAVYYCAAASGQVQ TVVVVTDRFYHYWGQGTQVTVSS | SEQID NO: 45 |
| Nucle ic acid seque nce | CAGGTGCAGCTGGTGGAGAGCGGCGGCGGCCTGGTGAAGCCCGGCGGCAGCCTGAGGC TGAGCTGCGCCGCCAGCGACGCCACCTACAGGAGGTACTGCATGGGCTGGTTCAGGCAG GCCCCCGGCAAGGAGAGGGAGAAGGTGGCCGCCATCTACACCGGCGACGCCACCACCTA CTACGCCGACAGCGTGAAGGGCAGGTTCACCATCAGCCAGGAGAACGCCAAGAACACCA TGTACCTGCAGATGAACAGCCTGAAGCCCGAGGACACCGCCATGTACTACTGCGCCGCCG ACAGGAGCGGCTGCAGCGTGGCCTTCAGCTACAGCAGCTTCGAGGACTGGGGCCAGGGC ACCCTGGTGACCGTGAGCAGCGGCGGCGGCGGCAGCGGCGGCGGCGGCAGCGGCGGCG GCGGCAGCCAGGTGCAGCTGGTGGAGAGCGGCGGCGGCCTGGTGCAGGCCGGCGGCAG CCTGAGGCTGAGCTGCGCCGCCAGCGGCAGGACCTTCAGCAGCTACGCCATGGGCTGGT TCAGGCAGGCCCCCGGCAAGGAGAGGGAGTTCGTGGCCGGCATCAGCTGGAGCGGCGA CAGCACCTACTACGCCGACAGCGTGAAGGGCAGGTTCACCATCAGCAGGGACAACGCCA AGAACACCGTGGGCCTGCAGATGAACAGCCTGAAGCCCGAGGACACCGCCGTGTACTAC TGCGCCGCCGCCAGCGGCCAGGTGCAGACCGTGGTGGTGGTGACCGACAGGTTCTACCA CTACTGGGGCCAGGGCACCCAGGTGACCGTGAGCAGC | SEQ ID NO: 46 |

### Example 6: Purification and expression of single-domain antibodies specifically against CD16a protein in Escherichia coli host bacteria

The plasmids (pMECS-VHH) of different clone strains obtained through sequencing analysis in Example 4 were electrotransformed into *Escherichia coli* HB2151 bacteria, and then the resulting bacteria were spread onto LB+amp+glucose plates, which were culture plates containing ampicillin and glucose, followed by culturing at 37°C overnight; single colonies were picked and inoculated into 5 mL of an LB liquid culture medium containing ampicillin and cultured on a shaker at 37°C overnight.

1 mL of bacterial strains that had been cultured overnight were inoculated into 330 mL of a TB liquid medium, and cultured on a shaker at 37°C until the OD600nm values reached 0.6-0.9, then 1M IPTG was added, followed by culturing on a shaker at 28°C overnight; *Escherichia coli* bacteria were collected by centrifugation, and a crude antibody extract was obtained by the osmotic burst method.

The single-domain antibodies were purified by nickel-column affinity chromatography.

### Example 7: Construction of eukaryotic expression vectors for Fc-Fusion antibodies of anti-CD16a single-domain antibody

(1) the sequences of interest obtained in Example 4 were subcloned into a eukaryotic expression vector: the antibodies screened out in Example 4 were subjected to Sanger sequencing to obtain their nucleotide sequences;
(2) the above nucleotide sequences were synthesized by sequence synthesis into the vector RJK-V4-hFC designed and modified in-house to obtain a recombinant eukaryotic expression vector, and the modification method for this vector was as described in Example 11;
(3) the recombinant eukaryotic expression vector constructed in step (2) was transformed into DH5α *Escherichia coli* bacteria and cultured, followed by plasmid extraction and endotoxin removal;
(4) the extracted plasmids were then subjected to sequencing for identification;
(5) the recombinant vector, once confirmed without any error, was prepared for subsequent transfection and expression in eukaryotic cells. The Fc protein of VHH was expressed using the method described in Example 8 or 9, and the resultant antibody was purified using the method described in Example 10.

### Example 8: Expression of single-domain antibodies against CD16a protein in ExpiCHO-S cells in suspension

(1) three days before transfection, ExpiCHO-S^{™} cells were passaged and expanded at 2.5×10⁵ cells/mL, and the cells in a calculated required volume were transferred into a 500 mL shake flask containing 120 mL (final volume) of a fresh pre-warmed ExpiCHO^{™} expression medium; the cells were adjusted to a concentration of about 4×10⁶-6×10⁶ viable cells/mL;
(2) one day before transfection, the ExpiCHO-S^{™} cells were diluted to a concentration of 3.5×10⁶ viable cells/mL, and cultured overnight;
(3) on the day of transfection, cell density and percentage of viable cells were determined. Before transfection, the cell density should reach approximately 7×10⁶-10×10⁶ viable cells/mL;
(4) the cells were diluted to 6×10⁶ viable cells/mL with the fresh ExpiCHO^{™} expression medium pre-warmed to 37°C. The cells in a calculated required volume were transferred into a 500 mL shake flask containing 100 mL (final volume) of the fresh pre-warmed ExpiCHO^{™} expression medium;
(5) ExpiFectamine^{™}CHO reagents were mixed evenly by gentle inversion, and 3.7 mL of the OptiPRO^{™} medium was used to dilute the ExpiFectamine^{™}CHO reagents, followed by vortexing or mixing uniformly;
(6) plasmid DNA was diluted with 4 mL of the refrigerated OptiPRO^{™} medium, followed by vortexing for mixing thoroughly;
(7) the ExpiFectamine CHO/plasmid DNA (the plasmid DNA being the eukaryotic expression vector for the Fc-fusion antibody of the anti-CD16a single-domain antibody prepared in Example 7) complex was incubated at room temperature for 1 to 5 minutes, and then gently added into the prepared cell suspension while lightly shaking the flask during the addition process;
(8) the cells were cultured with shaking in a humidified atmosphere of 8% CO₂ at 37°C;
(9) on day 1 after transfection (18-22 hours later), 600 µL of the ExpiFectamine^{™}CHO Enhancer and 24 mL of the ExpiCHO feed were added;
(10) approximately 8 days after transfection (when cell viability was below 70%), the supernatant was collected.

### Example 9: Expression of single-domain antibodies against CD16a protein in 293F cells in suspension

Experimental procedure for expression of recombinant single-domain antibodies (expression in a 500 mL shake flask was used as an example):
(1) three days before transfection, 293F cells were passaged and expanded at 2.5×10⁵ cells/mL, and the cells in a calculated required volume were transferred into a 500 mL shake flask containing 120 mL (final volume) of a fresh pre-warmed OPM-293 CD05 Medium. The cells were adjusted to a concentration of about 2×10⁶-3×10⁶ viable cells/mL.
(2) on the day of transfection, cell density and percentage of viable cells were determined. Before transfection, the cell density should reach approximately 2×10⁶-3×10⁶ viable cells/mL.
(3) the cells were diluted to 1×10⁶ viable cells/mL with the pre-warmed OPM-293 CD05 Medium. The cells in a calculated required volume were transferred into a 500 mL shake flask containing 100 mL (final volume) of the fresh and pre-warmed medium.
(4) the PEI (1 mg/mL) reagent was diluted with 4 mL of the Opti-MEM medium, followed by vortexing or pipetting to mix evenly; the plasmid DNA (which was the eukaryotic expression vector for the Fc-fusion antibody of the anti-CD16a single-domain antibody prepared in Example 7) was diluted with 4 mL of the Opt-MEM medium, followed by vortexing to mix thoroughly, and the resulting mixture was filtered through a 0.22 µm filter tip. The filtrate was incubated at room temperature for 5 minutes.
(5) the diluted PEI reagent was added to the diluted DNA, followed by mixing evenly by inversion. The PEI/plasmid DNA complex was incubated at room temperature for 15 to 20 minutes, and then gently added into the prepared cell suspension while the flask was gently swirled during the addition process.
(6) the cells were cultured with shaking at 120 rpm under a condition of 37°C and 5% CO₂.
(7) 5 mL of the OPM-CHO PFF05 feed was added at 24 hours and 72 hours after transfection.
(8) approximately 7 days after transfection (when cell viability was below 70%), the supernatant was collected.

### Example 10: Purification of single-domain antibodies against CD16a protein

(1) the protein expression supernatant obtained in Example 8 or 9 was filtered through a 0.45 µm disposable filter tip to remove insoluble impurities;
(2) the above filtrate was purified by affinity chromatography using a protein purifier, in which a Protein A-conjugated agarose filler was chosen for purification based on the capability of a human Fc to bind to Protein A;
(3) the filtrate was allowed to flow through a pre-packed protein A column at a flow rate of 1 mL/min, during which the protein of interest in the filtrate would bind to the filler;
(4) impurity proteins bound on the column were washed away with a low-salt buffer and a high-salt buffer;
(5) the protein of interest bound to the column was eluted with a low-pH buffer;
(6) a Tris-HCl solution at pH 9.0 was quickly added to the eluent for neutralization;
(7) the neutralized protein solution was dialyzed before subjected to SDS-PAGE analysis, and then stored at a low temperature for later use when it was determined that the protein purity was 95% or higher and the concentration was 0.5 mg/mL or higher.

### Example 11: Construction of eukaryotic expression vector RJK-V4-hFC for single-domain antibodies

The universal vector RJK-V4-hFC of interest for the mentioned nanobodies was an in-house modified vector based on the commercial vector pCDNA3.4 from Invitrogen (the link for the vector data: https://assets.thermofisher.com/TFS-Assets/LSG/manuals/pcdna3_4_topo_ta_cloning_kit_man.pdf), which was obtained by the fusion of an Fc segment in the human IgG1 heavy chain coding sequence therewith, in other words, this vector contained the IgG1 heavy chain hinge region, CH2, and CH3 regions. The specific modification protocol was as follows:
(1) the restriction enzyme cleavage sites XbaI and AgeI in pcDNA3.4 were selected;
(2) a multiple cloning site (MCS) and a 6×His tag were introduced at the 5' end and 3' end of the Fc fragment encoding sequence by overlapping PCR, respectively;
(3) the above fragment was amplified by PCR using a pair of primers respectively carrying the enzyme cleavage sites Xbal and AgeI;
(4) the pcDNA3.4 and the recombinant DNA fragment in (3) were cleaved using the restriction endonucleases XbaI and AgeI, respectively;
(5) the cleaved vector and insertion fragment were ligated with a T4 ligase, and the ligated product was then transformed into *Escherichia coli*, amplified, and sequenced for identification to obtain a recombinant plasmid.

### Example 12: Determination of antigen-binding dose-effect curves of antibodies

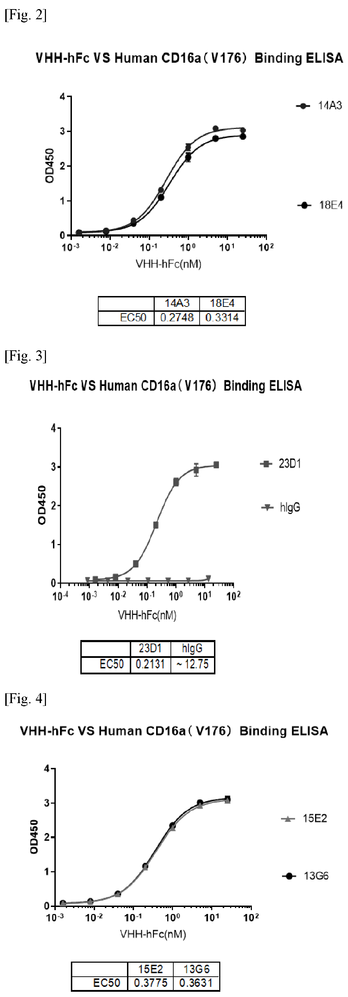

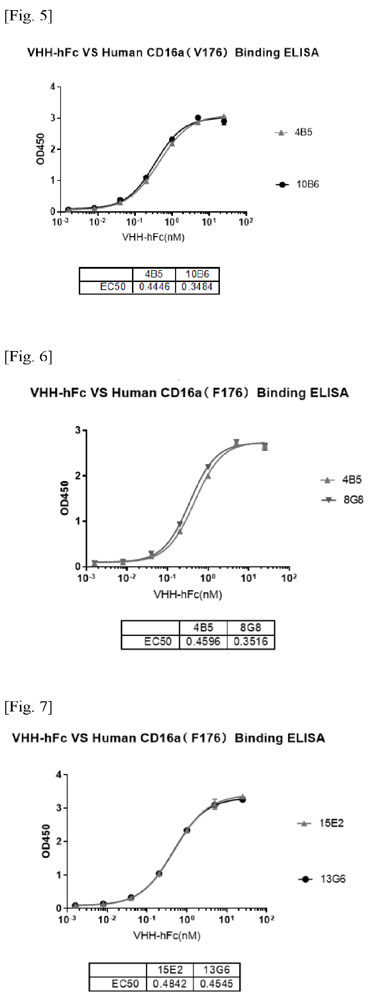

The enzyme-linked immunosorbent assay (ELISA) was conducted in this example according to the standard operating procedure.
(1) 50 µL of 1 µg/mL human CD16a-V176 or human CD16a-F176 protein was coated at 4°C overnight.
(2) the plates were washed; and 200 µL of 5% milk was added, followed by blocking at 37°C for 2 hours.
(3) the VHH-Fc was diluted to 2 µg/mL, and then the antibody was diluted in a 5-fold gradient, resulting in a total of 8 concentration gradients. The VHH-Fc mentioned herein refers to the Fc-fusion single-domain antibody obtained through purification in Example 10.
(4) the plates were washed; and 50 µL of the single-domain antibodies obtained through dilution in step (3) were added to each of two duplicate wells, followed by incubating at 37°C for 1 hour.
(5) the plates were washed; and 50 µL of HRP-Goat anti hIgG secondary antibody was added, followed by incubating at 37°C for 30 minutes.
(6) the plates were washed (repeated for several times); and 50 µL of TMB that had been restored to room temperature was added, followed by reaction at room temperature for 15 minutes in the dark.
(7) 50 µL of stop solution (1N HCl) was added, then values were read on a microplate reader and recorded.
(8) the curves were plotted and EC50s were calculated, as shown in Figures 2 to 9, where hIgG refers to an isotype control, an immunoglobulin molecule that would not bind to any target, and was commercially purchased. Figures 2-5 showed the binding dose-effect curves of the individual single-domain antibodies to human CD16a (V176), and Figures 6-9 showed the binding dose-effect curves of the individual single-domain antibodies to human CD16a (F176).

It could be seen from Figures 2-9 that the single-domain antibodies of the present invention all exhibited a favorable affinity and strong specificity to the CD16a protein.

### Example 13: Expression and purification of tool antibodies (Tabs)

The Tabs used in the examples of the present invention include: Tab1, 50NI, the sequence of which was from CN101583625B; Tab3, AFM13, the sequence of which was from CN110461357A; Tab4, AFM24, the sequence of which was from CN110461357A; and FOLR1-Tab1, farletuzumab.

The retrieved sequences by searching were entrusted to General Biosystems (Anhui) Co., Ltd. for codon optimization in mammalian cell expression systems, followed by cloning into the pcDNA3.1 vector. After resistance screening, plasmid-positive bacteria were selected for amplification, and the plasmids were extracted using a plasmid extraction midi kit (Macherey Nagel, Cat#740412.50). According to a ratio of 100 µg of plasmids (40 µg of heavy chain + 60 µg of light chain) per 100 mL of cells, the plasmids were added and transiently expressed in 293F cells using PEI (medium: FreeStyle 293 Expression medium, Thermo, Cat#12338026 + F-68, Thermo, Cat#24040032); after 6 to 24 hours of transfection, 10% Peptone (Sigma, Cat#P0521-100G) at 5% by volume was added, followed by culturing at 130 rpm in 8% CO₂ for about 7-8 days; the expression supernatant was collected when the cell viability dropped to 50%, and purified using the Protein A (GE, Cat#17-5438-02) gravity column; after dialysis against PBS, the concentration was determined using Nanodrop, the purity was identified by SEC, and the binding capacity was verified by indirect ELISA;

Tab1, Tab3, Tab4, and FOLR1-Tab1 obtained by this method had a concentration of not less than 2 mg/ml and a purity greater than 95%.

### Example 14: Detection of RKA effect of CD16a-hFc antibodies

The purpose of this example was to perform an RKA effect detection on the Fc-fusion single-domain antibodies specifically targeting CD16a, where the Fc-fusion single-domain antibodies involved were obtained through purification in Example 10, the cells used were P815 cells, and RKA refers to the Redirected Killing Assay. The steps for the experiment were as follows:
(1) P815 cells were collected by centrifugation.
(2) the P815 cells were resuspended in an Assay buffer (RPMI-1640+1%FBS), and the cell density was adjusted to 2×10⁵ cells/mL.
(3) 50 µL of the cell suspension was seeded into each well of a 96-well plate.
(4) Tab1 and the single-domain antibody samples to be tested were subjected to a 10-fold gradient dilution starting from 4×10 µg/mL.
(5) the gradient-diluted antibody solution was added to the cell suspension and incubated for 0.5 hours.
(6) PBMCs were collected by centrifugation, with the cell density adjusted to 2.5×10⁶ cells/mL, and 100 µL of the PBMC cell suspension was added to each well.
(7) the culture was incubated at 37°C and 5% CO₂ for 15 hours.
(8) the cell plate was centrifuged at 2000 rpm for 3 minutes, and 50 µL of the supernatant was transferred to a new 96-well plate.
(9) 50 µL of an LDH detection reagent was added into each well, and OD492 and OD650 were detected by using FlexStation 3.

The target cell killing rate (%) was calculated according to the following formula: Target cell killing rate (%) = (Sample - E/T) / (MAX - MIN);
based on the target cell killing rate and concentration, a four-parameter fitting was performed to calculate the EC₅₀ concentration of each antibody-mediated ADCC effect.

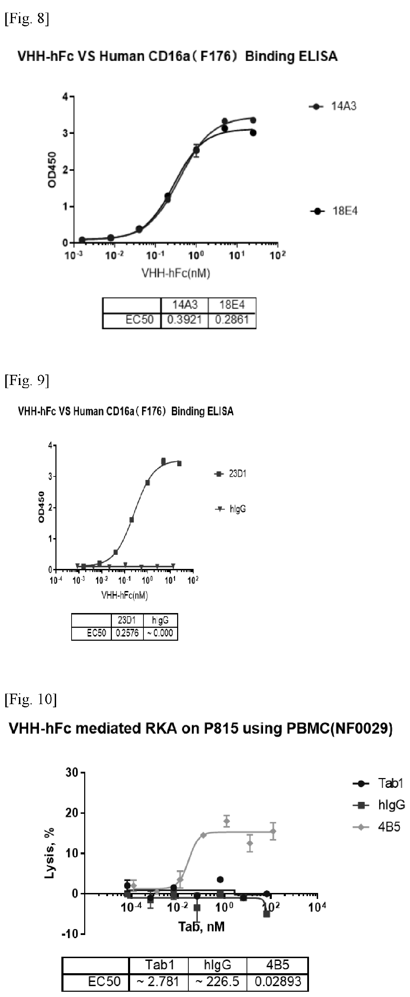

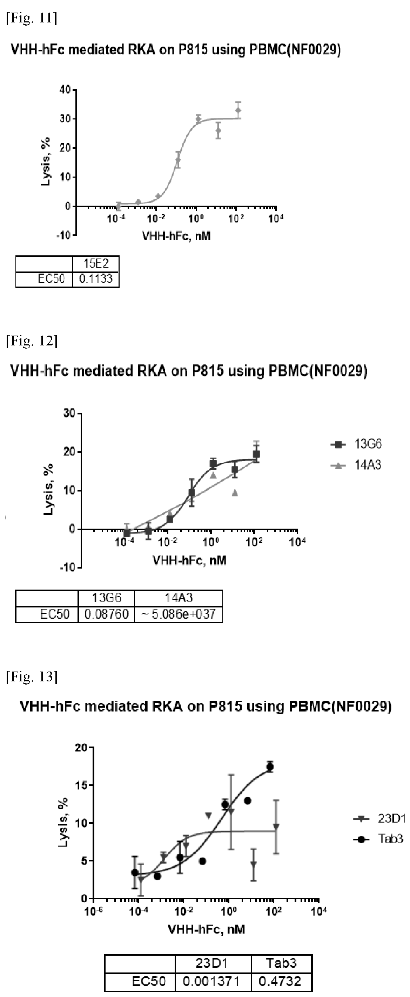

The results of the RKA effect detection were shown in Figures 10-13. It was evident that the CD16a single-domain antibodies of the present invention all have a strong redirected killing ability.

### Example 15: ADCC effect of CD16a-hFc antibodies

The ADCC effect of the CD16a-hFc of the present invention was determined using a reporter gene assay, with the following steps:
(1) 25 µL of an assay buffer was added into a 96-well plate.
(2) Tab, hIgG, and VHH-hFc samples were prepared into solutions with a maximum concentration of 10 µg/mL, and subjected to 10-fold gradient dilution to finally obtain 7 concentrations; the VHH-hFc was the Fc-fusion protein of the single-domain antibody against the CD16a protein prepared in Example 9 (expressed in 293F cells) and purified in Example 10.
(3) the above gradient-diluted antibody solutions were added to the cell culture wells in a volume equal to that of the cell suspension;
(4) for the sample wells and E/T wells (the antibody concentration was 0), Jurkat-NFAT-luc-FcγRIIIa cells were collected and added to the cell culture wells at a density of 20,000 cells/well;
(5) after 6 hours of incubation, cell killing was detected using the One-Glo kit and luminescence was read;
(6) the fold of induction was calculated according to the following formula: Fold of induction = (Sample - BG) / (E/T - BG).

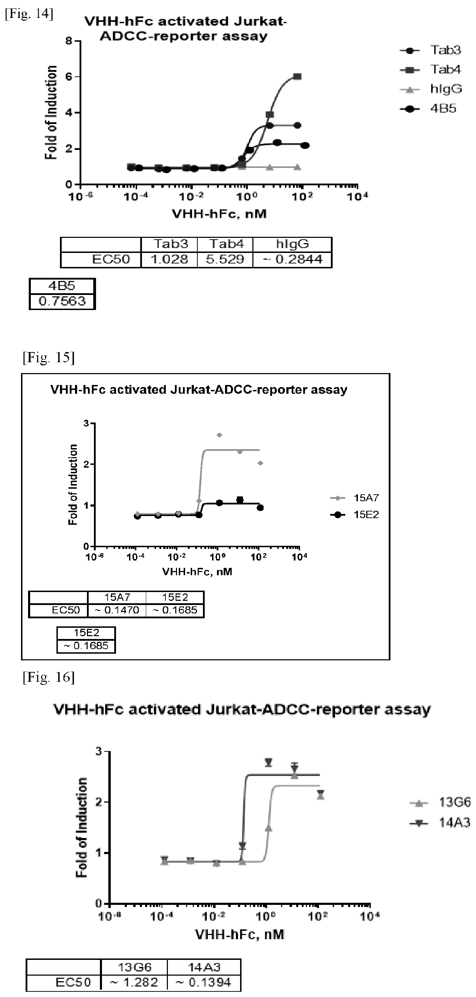

Based on the target cell killing rate and concentration, a four-parameter fitting was performed to calculate the EC₅₀ concentration of each antibody-mediated ADCC effect, as shown in Figures 14-17. As shown in Figures 14-17, all CD16a-hFc antibodies exhibited an ADCC effect.

### Example 16: Preparation of trispecific antibody FOLR1/CD16a/Nkp46

The preparation process of the trispecific antibody comprised the following steps:
(1) the nucleotide sequences in Table 18 were synthesized into pcDNA3.4 by sequence synthesis to obtain recombinant eukaryotic expression vectors;
(2) the recombinant eukaryotic expression vectors constructed in step (1) was transformed into DH5α Escherichia coli bacteria, and cultured, followed by plasmid extraction and endotoxin removal;
(3) the extracted plasmids were then subjected to sequencing for identification;
(4) the recombinant vectors, once confirmed without any error, were prepared for subsequent eukaryotic cell transfection and expression.

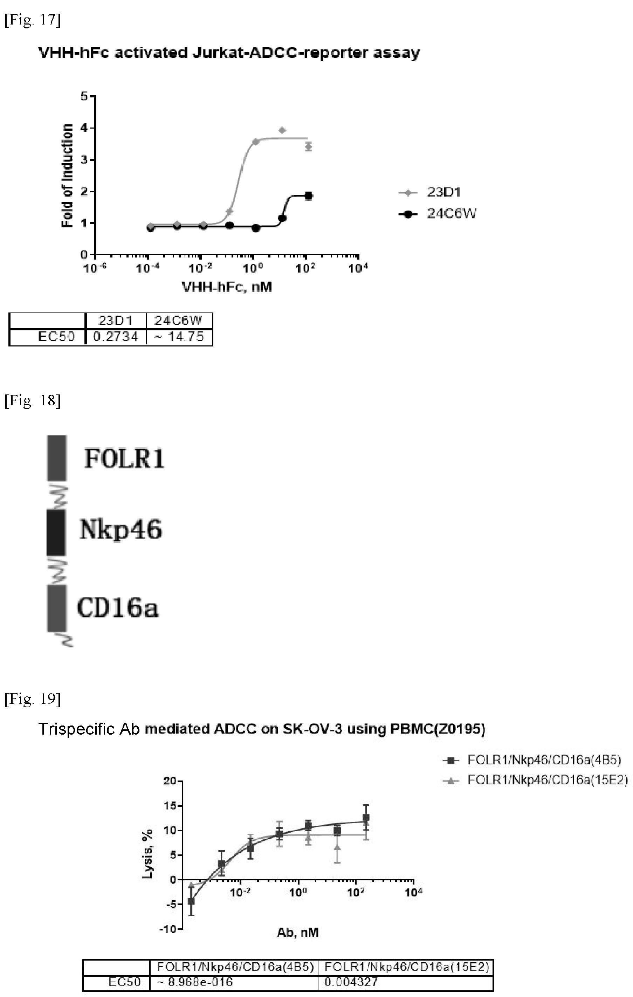

Trispecific antibodies were assembled from FOLR1/Nkp46 and anti-CD16a single-domain antibodies, with a structure shown in Figure 18 (FOLR1 VHH-linker-Nkp46 VHH-linker-CD16a VHH), and the amino acid sequences thereof were shown in SEQ ID NOs: 47-51. The trispecific antibodies were not additionally linked to an Fc.

In SEQ ID NOs: 47-51, the humanized 4F4 was shown as amino acids 1-125 therein, the linker between the humanized 4F4 and the humanized 7F10 was shown as amino acids 126-140 therein (GGGGSGGGGSGGGGS), the humanized 7F10 was shown as amino acids 141-267 therein, and the linker between the humanized 7F10 and each of the CD16a single-domain antibodies was shown as amino acids 268-282 therein (GGGGSGGGGSGGGGS).

All linkers in the present specification were not limited to specific sequences, and other linkers used for constructing antibodies in the prior art were all applicable.

In SEQ ID NO: 47, amino acids 283-408 represented the CD16a single-domain antibody 4B5. In SEQ ID NO: 48, amino acids 283-409 represented the CD16a single-domain antibody 15E2. In SEQ ID NO: 49, amino acids 283-409 represented the CD16a single-domain antibody 13G6. In SEQ ID NO: 50, amino acids 283-408 represented the CD16a single-domain antibody 14A3, and in SEQ ID NO: 51, amino acids 283-408 represented the CD16a single-domain antibody 23D1.

**Table 17 Amino acid sequences of the trispecific antibodies**

| | Sequence | SEQ ID |
|---|---|---|
| FOLR1/ Nkp46/ CD16a (4B5) | QVQLVESGGGLVKPGGSLRLSCAASDATYRRYCMGWFRQAPGKEREKVAAIYTGDA TTYYADSVKGRFTISQENAKNTMYLQMNSLKPEDTAMYYCAADRSGCSVAFSYSSFE DWGQGTLVTVSSGGGGSGGGGSGGGGSQVQLVESGGGLVQAGGSLRLSCAASGRTF SSYAMGWFRQAPGKEREFVAGISWSGDSTYYADSVKGRFTISRDNAKNTVGLQMNSL KPEDTAVYYCAAASGQVQTVVVVTDRFYHYWGQGTQVTVSSGGGGSGGGGSGGGG SQVQLVESGGGLVQAGGSLRLSCAASGGTFRIYAMGWFRQAPGKEREFVGSISMMRG ITDYAESVKGRFTISRDNAKFTMYLQMNSLKSEDTAVYYCAKAVDKSPYGGWGWGP YDDWGQGTQVTVSS | SEQ ID NO: 47 |
| FOLR1/ Nkp46/ CD16a (15E2) | QVQLVESGGGLVKPGGSLRLSCAASDATYRRYCMGWFRQAPGKEREKVAAIYTGDA TTYYADSVKGRFTISQENAKNTMYLQMNSLKPEDTAMYYCAADRSGCSVAFSYSSFE DWGQGTLVTVSSGGGGSGGGGSGGGGSQVQLVESGGGLVQAGGSLRLSCAASGRTF SSYAMGWFRQAPGKEREFVAGISWSGDSTYYADSVKGRFTISRDNAKNTVGLQMNSL KPEDTAVYYCAAASGQVQTVVVVTDRFYHYWGQGTQVTVSSGGGGSGGGGSGGGG SQVQLVESGGGLVQAGDSLRLSCAASGGTFRIYAMGWFRQAPGKEREFVGSISMMRG ITDYAESVKGRFTISRDNAKFTMYLQMNTLKPEDTAVYYCAIRERPPPMGWGLAGISY DYWGQGTQVTVSS | SEQ ID NO: 48 |
| FOLR1/ Nkp46/ CD16a (13G6) | QVQLVESGGGLVKPGGSLRLSCAASDATYRRYCMGWFRQAPGKEREKVAAIYTGDA TTYYADSVKGRFTISQENAKNTMYLQMNSLKPEDTAMYYCAADRSGCSVAFSYSSFE DWGQGTLVTVSSGGGGSGGGGSGGGGSQVQLVESGGGLVQAGGSLRLSCAASGRTF SSYAMGWFRQAPGKEREFVAGISWSGDSTYYADSVKGRFTISRDNAKNTVGLQMNSL KPEDTAVYYCAAASGQVQTVVVVTDRFYHYWGQGTQVTVSSGGGGSGGGGSGGGG SQVQLVESGGGLVQPGGSLRLSCAASGRTFRIYAMGWFRQAPGKEREFVGSIRLMRGI TDYADSVKGRFTISRDNAKFTMYLQMNTLKPEDTAVYYCAAVGSGGDFYTPSTSGEY RYWGQGTQVTVSS | SEQ ID NO: 49 |
| FOLR1/ Nkp46/ CD16a (14A3) | QVQLVESGGGLVKPGGSLRLSCAASDATYRRYCMGWFRQAPGKEREKVAAIYTGDA TTYYADSVKGRFTISQENAKNTMYLQMNSLKPEDTAMYYCAADRSGCSVAFSYSSFE DWGQGTLVTVSSGGGGSGGGGSGGGGSQVQLVESGGGLVQAGGSLRLSCAASGRTF SSYAMGWFRQAPGKEREFVAGISWSGDSTYYADSVKGRFTISRDNAKNTVGLQMNSL KPEDTAVYYCAAASGQVQTVVVVTDRFYHYWGQGTQVTVSSGGGGSGGGGSGGGG SQVQLVESGGGLVQPGGSLRLSCEVSGRTFSYLALGWFRQAPGKEREFVAAASWGGG STGYADSVKGRFAISRDNVKNTQYLQMNSLKPEDTAVYYCVVGRRFKGSVYYTPNE YEYWGQGTQVTVSS | SEQ ID NO: 50 |
| FOLR1/ Nkp46/ CD16a (23D1) | QVQLVESGGGLVKPGGSLRLSCAASDATYRRYCMGWFRQAPGKEREKVAAIYTGDA TTYYADSVKGRFTISQENAKNTMYLQMNSLKPEDTAMYYCAADRSGCSVAFSYSSFE DWGQGTLVTVSSGGGGSGGGGSGGGGSQVQLVESGGGLVQAGGSLRLSCAASGRTF SSYAMGWFRQAPGKEREFVAGISWSGDSTYYADSVKGRFTISRDNAKNTVGLQMNSL KPEDTAVYYCAAASGQVQTVVVVTDRFYHYWGQGTQVTVSSGGGGSGGGGSGGGG SQVQLVESGGGLVQAGGSLRLSCAVSGRTFSYLAMGWFRQAPGKEREFVAASSWGG GTSGYADSVKGRFAISRDNAKNTQYLQMNSLKPEDTAVYYCVVGRRFKGSVYYTPN EYEYWGQGTQVTVSS | SEQ ID NO: 51 |

**Table 18 Nucleic acid sequences of the trispecific antibodies**

| | Sequence | SEQ ID |
|---|---|---|
| FOLR1/ Nkp46/ CD16a (4B5) | CAGGTGCAGCTGGTGGAGAGCGGCGGCGGCCTGGTGAAGCCCGGCGGCAGCCT GAGGCTGAGCTGCGCCGCCAGCGACGCCACCTACAGGAGGTACTGCATGGGCT GGTTCAGGCAGGCCCCCGGCAAGGAGAGGGAGAAGGTGGCCGCCATCTACACC GGCGACGCCACCACCTACTACGCCGACAGCGTGAAGGGCAGGTTCACCATCAG CCAGGAGAACGCCAAGAACACCATGTACCTGCAGATGAACAGCCTGAAGCCCG AGGACACCGCCATGTACTACTGCGCCGCCGACAGGAGCGGCTGCAGCGTGGCCT TCAGCTACAGCAGCTTCGAGGACTGGGGCCAGGGCACCCTGGTGACCGTGAGC AGCGGCGGCGGCGGCAGCGGCGGCGGCGGCAGCGGCGGCGGCGGCAGCCAGG TGCAGCTGGTGGAGAGCGGCGGCGGCCTGGTGCAGGCCGGCGGCAGCCTGAGG CTGAGCTGCGCCGCCAGCGGCAGGACCTTCAGCAGCTACGCCATGGGCTGGTTC AGGCAGGCCCCCGGCAAGGAGAGGGAGTTCGTGGCCGGCATCAGCTGGAGCGG CGACAGCACCTACTACGCCGACAGCGTGAAGGGCAGGTTCACCATCAGCAGGG ACAACGCCAAGAACACCGTGGGCCTGCAGATGAACAGCCTGAAGCCCGAGGAC ACCGCCGTGTACTACTGCGCCGCCGCCAGCGGCCAGGTGCAGACCGTGGTGGTG GTGACCGACAGGTTCTACCACTACTGGGGCCAGGGCACCCAGGTGACCGTGAGC AGCGGCGGCGGCGGCAGCGGCGGCGGCGGCAGCGGCGGCGGCGGCAGCCAGG TGCAGCTGGTGGAGAGCGGCGGCGGCCTGGTGCAGGCCGGCGGCAGCCTGAGG CTGAGCTGCGCCGCCAGCGGCGGCACCTTCAGGATCTACGCCATGGGCTGGTTC AGGCAGGCCCCCGGCAAGGAGAGGGAGTTCGTGGGCAGCATCAGCATGATGAG GGGCATCACCGACTACGCCGAGAGCGTGAAGGGCAGGTTCACCATCAGCAGGG ACAACGCCAAGTTCACCATGTACCTGCAGATGAACAGCCTGAAGAGCGAGGAC ACCGCCGTGTACTACTGCGCCAAGGCCGTGGACAAGAGCCCCTACGGCGGCTGG GGCTGGGGCCCCTACGACGACTGGGGCCAGGGCACCCAGGTGACCGTGAGCAG C | SEQ ID NO: 52 |
| FOLR1/ Nkp46/ CD16a (15E2) | CAGGTGCAGCTGGTGGAGAGCGGCGGCGGCCTGGTGAAGCCCGGCGGCAGCCT GAGGCTGAGCTGCGCCGCCAGCGACGCCACCTACAGGAGGTACTGCATGGGCT GGTTCAGGCAGGCCCCCGGCAAGGAGAGGGAGAAGGTGGCCGCCATCTACACC GGCGACGCCACCACCTACTACGCCGACAGCGTGAAGGGCAGGTTCACCATCAG CCAGGAGAACGCCAAGAACACCATGTACCTGCAGATGAACAGCCTGAAGCCCG AGGACACCGCCATGTACTACTGCGCCGCCGACAGGAGCGGCTGCAGCGTGGCCT TCAGCTACAGCAGCTTCGAGGACTGGGGCCAGGGCACCCTGGTGACCGTGAGC AGCGGCGGCGGCGGCAGCGGCGGCGGCGGCAGCGGCGGCGGCGGCAGCCAGG TGCAGCTGGTGGAGAGCGGCGGCGGCCTGGTGCAGGCCGGCGGCAGCCTGAGG CTGAGCTGCGCCGCCAGCGGCAGGACCTTCAGCAGCTACGCCATGGGCTGGTTC AGGCAGGCCCCCGGCAAGGAGAGGGAGTTCGTGGCCGGCATCAGCTGGAGCGG CGACAGCACCTACTACGCCGACAGCGTGAAGGGCAGGTTCACCATCAGCAGGG ACAACGCCAAGAACACCGTGGGCCTGCAGATGAACAGCCTGAAGCCCGAGGAC ACCGCCGTGTACTACTGCGCCGCCGCCAGCGGCCAGGTGCAGACCGTGGTGGTG GTGACCGACAGGTTCTACCACTACTGGGGCCAGGGCACCCAGGTGACCGTGAGC AGCGGCGGCGGCGGCAGCGGCGGCGGCGGCAGCGGCGGCGGCGGCAGCCAGG TGCAGCTGGTGGAGAGCGGCGGCGGCCTGGTGCAGGCCGGCGACAGCCTGAGG CTGAGCTGCGCCGCCAGCGGCGGCACCTTCAGGATCTACGCCATGGGCTGGTTC AGGCAGGCCCCCGGCAAGGAGAGGGAGTTCGTGGGCAGCATCAGCATGATGAG GGGCATCACCGACTACGCCGAGAGCGTGAAGGGCAGGTTCACCATCAGCAGGG ACAACGCCAAGTTCACCATGTACCTGCAGATGAACACCCTGAAGCCCGAGGAC ACCGCCGTGTACTACTGCGCCATCAGGGAGAGGCCCCCCCCCATGGGCTGGGGC CTGGCCGGCATCAGCTACGACTACTGGGGCCAGGGCACCCAGGTGACCGTGAG CAGC | SEQ ID NO: 53 |
| FOLR1/ Nkp46/ CD16a (13G6) | CAGGTGCAGCTGGTGGAGAGCGGCGGCGGCCTGGTGAAGCCCGGCGGCAGCCT GAGGCTGAGCTGCGCCGCCAGCGACGCCACCTACAGGAGGTACTGCATGGGCT GGTTCAGGCAGGCCCCCGGCAAGGAGAGGGAGAAGGTGGCCGCCATCTACACC GGCGACGCCACCACCTACTACGCCGACAGCGTGAAGGGCAGGTTCACCATCAG CCAGGAGAACGCCAAGAACACCATGTACCTGCAGATGAACAGCCTGAAGCCCG AGGACACCGCCATGTACTACTGCGCCGCCGACAGGAGCGGCTGCAGCGTGGCCT TCAGCTACAGCAGCTTCGAGGACTGGGGCCAGGGCACCCTGGTGACCGTGAGC AGCGGCGGCGGCGGCAGCGGCGGCGGCGGCAGCGGCGGCGGCGGCAGCCAGG TGCAGCTGGTGGAGAGCGGCGGCGGCCTGGTGCAGGCCGGCGGCAGCCTGAGG CTGAGCTGCGCCGCCAGCGGCAGGACCTTCAGCAGCTACGCCATGGGCTGGTTC AGGCAGGCCCCCGGCAAGGAGAGGGAGTTCGTGGCCGGCATCAGCTGGAGCGG CGACAGCACCTACTACGCCGACAGCGTGAAGGGCAGGTTCACCATCAGCAGGG ACAACGCCAAGAACACCGTGGGCCTGCAGATGAACAGCCTGAAGCCCGAGGAC ACCGCCGTGTACTACTGCGCCGCCGCCAGCGGCCAGGTGCAGACCGTGGTGGTG GTGACCGACAGGTTCTACCACTACTGGGGCCAGGGCACCCAGGTGACCGTGAGC AGCGGCGGCGGCGGCAGCGGCGGCGGCGGCAGCGGCGGCGGCGGCAGCCAGG TGCAGCTGGTGGAGAGCGGCGGCGGCCTGGTGCAGCCCGGCGGCAGCCTGAGG CTGAGCTGCGCCGCCAGCGGCAGGACCTTCAGGATCTACGCCATGGGCTGGTTC AGGCAGGCCCCCGGCAAGGAGAGGGAGTTCGTGGGCAGCATCAGGCTGATGAG GGGCATCACCGACTACGCCGACAGCGTGAAGGGCAGGTTCACCATCAGCAGGG ACAACGCCAAGTTCACCATGTACCTGCAGATGAACACCCTGAAGCCCGAGGAC ACCGCCGTGTACTACTGCGCCGCCGTGGGCAGCGGCGGCGACTTCTACACCCCC AGCACCAGCGGCGAGTACAGGTACTGGGGCCAGGGCACCCAGGTGACCGTGAG CAGC | SEQ ID NO: 54 |
| FOLR1/ Nkp46/ CD16a (14A3) | CAGGTGCAGCTGGTGGAGAGCGGCGGCGGCCTGGTGAAGCCCGGCGGCAGCCT GAGGCTGAGCTGCGCCGCCAGCGACGCCACCTACAGGAGGTACTGCATGGGCT GGTTCAGGCAGGCCCCCGGCAAGGAGAGGGAGAAGGTGGCCGCCATCTACACC GGCGACGCCACCACCTACTACGCCGACAGCGTGAAGGGCAGGTTCACCATCAG CCAGGAGAACGCCAAGAACACCATGTACCTGCAGATGAACAGCCTGAAGCCCG AGGACACCGCCATGTACTACTGCGCCGCCGACAGGAGCGGCTGCAGCGTGGCCT TCAGCTACAGCAGCTTCGAGGACTGGGGCCAGGGCACCCTGGTGACCGTGAGC AGCGGCGGCGGCGGCAGCGGCGGCGGCGGCAGCGGCGGCGGCGGCAGCCAGG TGCAGCTGGTGGAGAGCGGCGGCGGCCTGGTGCAGGCCGGCGGCAGCCTGAGG CTGAGCTGCGCCGCCAGCGGCAGGACCTTCAGCAGCTACGCCATGGGCTGGTTC AGGCAGGCCCCCGGCAAGGAGAGGGAGTTCGTGGCCGGCATCAGCTGGAGCGG CGACAGCACCTACTACGCCGACAGCGTGAAGGGCAGGTTCACCATCAGCAGGG ACAACGCCAAGAACACCGTGGGCCTGCAGATGAACAGCCTGAAGCCCGAGGAC ACCGCCGTGTACTACTGCGCCGCCGCCAGCGGCCAGGTGCAGACCGTGGTGGTG GTGACCGACAGGTTCTACCACTACTGGGGCCAGGGCACCCAGGTGACCGTGAGC AGCGGCGGCGGCGGCAGCGGCGGCGGCGGCAGCGGCGGCGGCGGCAGCCAGG TGCAGCTGGTGGAGAGCGGCGGCGGCCTGGTGCAGCCCGGCGGCAGCCTGAGG CTGAGCTGCGAGGTGAGCGGCAGGACCTTCAGCTACCTGGCCCTGGGCTGGTTC AGGCAGGCCCCCGGCAAGGAGAGGGAGTTCGTGGCCGCCGCCAGCTGGGGCGG CGGCAGCACCGGCTACGCCGACAGCGTGAAGGGCAGGTTCGCCATCAGCAGGG ACAACGTGAAGAACACCCAGTACCTGCAGATGAACAGCCTGAAGCCCGAGGAC ACCGCCGTGTACTACTGCGTGGTGGGCAGGAGGTTCAAGGGCAGCGTGTACTAC ACCCCCAACGAGTACGAGTACTGGGGCCAGGGCACCCAGGTGACCGTGAGCAG C | SEQ ID NO: 55 |
| FOLR1/ Nkp46/ CD16a (23D1) | CAGGTGCAGCTGGTGGAGAGCGGCGGCGGCCTGGTGAAGCCCGGCGGCAGCCT GAGGCTGAGCTGCGCCGCCAGCGACGCCACCTACAGGAGGTACTGCATGGGCT GGTTCAGGCAGGCCCCCGGCAAGGAGAGGGAGAAGGTGGCCGCCATCTACACC GGCGACGCCACCACCTACTACGCCGACAGCGTGAAGGGCAGGTTCACCATCAG CCAGGAGAACGCCAAGAACACCATGTACCTGCAGATGAACAGCCTGAAGCCCG AGGACACCGCCATGTACTACTGCGCCGCCGACAGGAGCGGCTGCAGCGTGGCCT TCAGCTACAGCAGCTTCGAGGACTGGGGCCAGGGCACCCTGGTGACCGTGAGC AGCGGCGGCGGCGGCAGCGGCGGCGGCGGCAGCGGCGGCGGCGGCAGCCAGG TGCAGCTGGTGGAGAGCGGCGGCGGCCTGGTGCAGGCCGGCGGCAGCCTGAGG CTGAGCTGCGCCGCCAGCGGCAGGACCTTCAGCAGCTACGCCATGGGCTGGTTC AGGCAGGCCCCCGGCAAGGAGAGGGAGTTCGTGGCCGGCATCAGCTGGAGCGG CGACAGCACCTACTACGCCGACAGCGTGAAGGGCAGGTTCACCATCAGCAGGG ACAACGCCAAGAACACCGTGGGCCTGCAGATGAACAGCCTGAAGCCCGAGGAC ACCGCCGTGTACTACTGCGCCGCCGCCAGCGGCCAGGTGCAGACCGTGGTGGTG GTGACCGACAGGTTCTACCACTACTGGGGCCAGGGCACCCAGGTGACCGTGAGC AGCGGCGGCGGCGGCAGCGGCGGCGGCGGCAGCGGCGGCGGCGGCAGCCAGG TGCAGCTGGTGGAGAGCGGCGGCGGCCTGGTGCAGGCCGGCGGCAGCCTGAGG CTGAGCTGCGCCGTGAGCGGCAGGACCTTCAGCTACCTGGCCATGGGCTGGTTC AGGCAGGCCCCCGGCAAGGAGAGGGAGTTCGTGGCCGCCAGCAGCTGGGGCGG CGGCACCAGCGGCTACGCCGACAGCGTGAAGGGCAGGTTCGCCATCAGCAGGG ACAACGCCAAGAACACCCAGTACCTGCAGATGAACAGCCTGAAGCCCGAGGAC ACCGCCGTGTACTACTGCGTGGTGGGCAGGAGGTTCAAGGGCAGCGTGTACTAC ACCCCCAACGAGTACGAGTACTGGGGCCAGGGCACCCAGGTGACCGTGAGCAG C | SEQ ID NO: 56 |

### Example 17: ADCC effect of FOLR1/Nkp46/CD16a trispecific antibodies

The ADCC effect of the trispecific antibodies of the present invention was determined using the LDH assay and the steps were as follows:
(1) SK-OV-3 cells, which had been passaged for 3-4 generations after recovery, were collected and seeded into a 96-well plate at a density of 10,000 cells/well;
(2) Tab, hIgG, FOLR1/Nkp46, and the trispecific antibodies were prepared into solutions with a maximum concentration of 10 µg/mL, and subjected to 10-fold gradient dilution to finally obtain 7 concentrations;
(3) the above gradient-diluted antibody solutions were added to the cell culture wells in a volume equal to that of the cell suspension;
(4) for the sample wells and E/T wells (the antibody concentration was 0), PBMC cells were collected and added to the cell culture wells at a density of 250,000 cells/well and in a volume twice that of a target cell suspension; for MAX wells, a lysis buffer was added to each well in a volume twice that of the target cell suspension; for MIN wells, an assay buffer was added to each well in a volume twice that of the target cell suspension;
(5) after 6 hours of incubation, cell killing was detected using an LDH kit and the absorbance was read;
(6) the target cell killing rate (%) was calculated according to the following formula: Target cell killing rate (%) = (Sample - E/T) / (MAX - MIN);
(7) based on the target cell killing rate and concentration, a four-parameter fitting was performed to calculate the EC₅₀ concentration of each antibody-mediated ADCC effect.

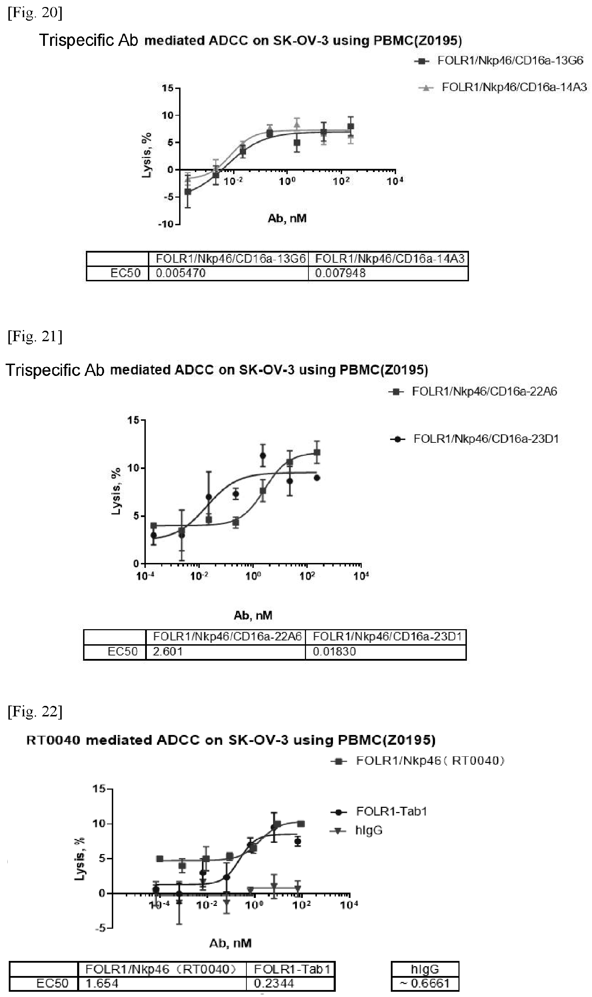

Based on the target cell killing rate and concentration, a four-parameter fitting was performed to calculate the EC₅₀ concentration of each antibody-mediated ADCC effect, as shown in Figures 19-22. It could be seen from Figures 19-22 that all trispecific antibodies exhibited a favorable ADCC effect.

The basic principles, main features, and advantages of the present invention have been shown and described above. It should be understood by those skilled in the art that the above examples are not intended to limit the present invention in any way, and all technical solutions obtained by equivalent substitution or equivalent transformation shall fall within the protection scope of the present disclosure.

## Claims

1. An anti-CD16a single-domain antibody, **characterized in that**: the single-domain antibody is composed of a heavy chain, comprising a heavy chain CDR1 as set forth in any one of SEQ ID NO: 11-SEQ ID NO: 13, a heavy chain CDR2 as set forth in any one of SEQ ID NO: 14-SEQ ID NO: 17, and a heavy chain CDR3 as set forth in any one of SEQ ID NO: 18-SEQ ID NO: 21.

2. The anti-CD16a single-domain antibody according to claim 1, **characterized in that**: the amino acid sequences of the heavy chain CDR1, heavy chain CDR2, and heavy chain CDR3 are one of the following (1)-(5):
(1) the CDR1 as set forth in SEQ ID NO: 12, the CDR2 as set forth in SEQ ID NO: 17, and the CDR3 as set forth in SEQ ID NO: 21;
(2) the CDR1 as set forth in SEQ ID NO: 12, the CDR2 as set forth in SEQ ID NO: 14, and the CDR3 as set forth in SEQ ID NO: 21;
(3) the CDR1 as set forth in SEQ ID NO: 11, the CDR2 as set forth in SEQ ID NO: 16, and the CDR3 as set forth in SEQ ID NO: 20;
(4) the CDR1 as set forth in SEQ ID NO: 11, the CDR2 as set forth in SEQ ID NO: 16, and the CDR3 as set forth in SEQ ID NO: 19; and
(5) the CDR1 as set forth in SEQ ID NO: 13, the CDR2 as set forth in SEQ ID NO: 15, and the CDR3 as set forth in SEQ ID NO: 18.

3. The anti-CD16a single-domain antibody according to claim 1, **characterized in that**: the single-domain antibody further comprises a framework region FR; wherein the framework region FR comprises amino acid sequences of an FR1, an FR2, an FR3, and an FR4; and the amino acid sequences of the framework region FR are respectively as follows:
the FR1 as set forth in any one of SEQ ID NOs: 22-26 or an FR1 variant thereof, wherein the FR1 variant comprises at most 5 amino acid substitutions in the FR1;
the FR2 as set forth in any one of SEQ ID NOs: 27-29 or an FR2 variant thereof, wherein the FR2 variant comprises at most 5 amino acid substitutions in the FR2;
the FR3 as set forth in any one of SEQ ID NOs: 30-34 or an FR3 variant thereof, wherein the FR3 variant comprises at most 5 amino acid substitutions in the FR3; and
the FR4 as set forth in SEQ ID NO: 35 or an FR4 variant thereof, wherein the FR4 variant comprises at most 5 amino acid substitutions in the FR4.

4. An anti-CD16a single-domain antibody, **characterized in that**: the amino acid sequence of the single-domain antibody is set forth in any one of SEQ ID NOs: 1-5, or the amino acid sequence of the single-domain antibody has at least one amino acid residue in the FR1, FR2, FR3 or FR4 sequence replaced by a conservative amino acid compared with any one of SEQ ID NOs: 1-5.

5. An Fc-fusion antibody or a humanized antibody of the anti-CD16a single-domain antibody according to any one of claims 1 to 4.

6. A recombinant protein, **characterized in that** the recombinant protein comprises the anti-CD16a single-domain antibody according to any one of claims 1 to 4.

7. A bispecific antibody or a multispecific antibody, **characterized by** comprising the single-domain antibody according to any one of claims 1 to 4, wherein the single-domain antibody serves as a first antigen-binding moiety that specifically binds to CD16a.

8. The bispecific antibody or multispecific antibody according to claim 7, **characterized by** further comprising a binding moiety specific for a different tumor antigen other than CD16a;
preferably, the different tumor antigen other than CD16a includes FOLR1, Her2, DEC205, CLEC9A, CEACAM5, CTLA4, CD3, CD7, CD11c, CD19, CD20, CD22, CD40, CD44, CD206, EGFR, EGFRvIII, Fibroblast activation protein (FAP), CA9, MMP-2, PD-L1, SIRPa, Trop2, GPC1, GPC3, cMET, VEGFR, Claudin18.2, Nkp46, CD30, NKG2D, IL-2Rβ, BCMA, CD123, TGF-β, CD38, IL-7, IL-8, FRα, NCR3, IL-15, Muc1, IL-16 or any other tumor antigen.

9. The bispecific antibody or multispecific antibody according to claim 7, **characterized in that** the bispecific antibody includes bispecific antibodies against FOLR1/CD16a, Her2/CD16a, DEC205/CD16a, CLEC9A/CD16a, CEACAM5/CD16a, CTLA4/CD16a, CD3/CD16a, CD7/CD16a, CD11c/CD16a, CD19/CD16a, CD20/CD16a, CD22/CD16a, CD40/CD16a, CD44/CD16a, CD206/CD16a, EGFR/CD16a, EGFRvIII/CD16a, Fibroblast activation protein (FAP)/CD16a, CA9/CD16a, MMP-2/CD16a, PD-L1/CD16a, SIRPa/CD16a, Trop2/CD16a, GPC1/CD16a, GPC3/CD16a, cMET/CD16a, VEGFR/CD16a, Claudin18.2/CD16a, CD30/CD16a, NKG2D/CD16a, IL-2Rβ/CD16a, BCMA/CD16a, CD123/CD16a, TGF-β/CD16a, CD38/CD16a, IL-7/CD16a, IL-8/CD16a, FRα/CD16a, NCR3/CD16a, IL-15/CD16a, Muc1/CD16a or IL-16/CD16a.

10. The bispecific antibody or multispecific antibody according to claim 7, wherein the multispecific antibody is a trispecific antibody, comprising a first antigen-binding moiety that specifically binds to CD16a, a second antigen-binding moiety that specifically binds to Nkp46, and a third antigen-binding moiety that specifically binds to a different tumor antigen other than CD16a and Nkp46;
the trispecific antibody includes those against FOLR1/CD16a/Nkp46, Her2/CD16a/Nkp46, DEC205/CD16a/Nkp46, CLEC9A/CD16a/Nkp46, CEACAM5/CD16a/Nkp46, CTLA4/CD16a/Nkp46, CD3/CD16a/Nkp46, CD7/CD16a/Nkp46, CD11c/CD16a/Nkp46, CD19/CD16a/Nkp46, CD20/CD16a/Nkp46, CD22/CD16a/Nkp46, CD40/CD16a/Nkp46, CD44/CD16a/Nkp46, CD206/CD16a/Nkp46, EGFR/CD16a/Nkp46, EGFRvIII/CD16a/Nkp46, Fibroblast activation protein (FAP)/CD16a/Nkp46, CA9/CD16a/Nkp46, MMP-2/CD16a/Nkp46, PD-L1/CD16a/Nkp46, SIRPa/CD16a/Nkp46, Trop2/CD16a/Nkp46, GPC1/CD16a/Nkp46, GPC3/CD16a/Nkp46, cMET/CD16a/Nkp46, VEGFR/CD16a/Nkp46, Claudin18.2/CD16a/Nkp46, CD30/CD16a/Nkp46, NKG2D/CD16a/Nkp46, IL-2Rβ/CD16a/Nkp46, BCMA/CD16a/Nkp46, CD123/CD16a/Nkp46, TGF-β/CD16a/Nkp46, CD38/CD16a/Nkp46, IL-7/CD16a/Nkp46, IL-8/CD16a/Nkp46, FRα/CD16a/Nkp46, NCR3/CD16a/Nkp46, IL-15/CD16a/Nkp46, Muc1/CD16a/Nkp46 or IL-16/CD16a/Nkp46.

11. The bispecific antibody or multispecific antibody according to claim 7, wherein the multispecific antibody is a trispecific antibody, comprising a first antigen-binding moiety that specifically binds to CD16a, a second antigen-binding moiety that specifically binds to Nkp46, and a third antigen-binding moiety that specifically binds to FOLR1;
the second antigen-binding moiety has the CDR1 as set forth in SEQ ID NO: 63, the CDR2 as set forth in SEQ ID NO: 65, and the CDR3 as set forth in SEQ ID NO: 70;
the third antigen-binding moiety has the CDR1 as set forth in SEQ ID NO: 58, the CDR2 as set forth in SEQ ID NO: 59, and the CDR3 as set forth in SEQ ID NO: 60;
preferably, the second antigen-binding moiety that specifically binds to Nkp46 and the third antigen-binding moiety that specifically binds to FOLR1 are VHHs;
preferably, the trispecific antibody is not fused to an Fc.

12. The bispecific antibody or multispecific antibody according to claim 10, **characterized in that** the amino acid sequences of the trispecific antibody are set forth in SEQ ID NOs: 47-51, respectively.

13. A polynucleotide molecule encoding the anti-CD16a single-domain antibody according to any one of claims 1-4, **characterized in that**: the nucleotide sequence thereof is set forth in any one of SEQ ID NOs: 6-10, or the amino acid sequence encoded by the nucleotide sequence is identical to the amino acid sequence encoded by any one of SEQ ID NOs: 6-10.

14. A polynucleotide molecule encoding the bispecific antibody or the multispecific antibody according to any one of claims 7-12, **characterized in that**: the nucleotide sequence thereof is set forth in any one of SEQ ID NOs: 52-56, or the amino acid sequence encoded by the nucleotide sequence is identical to the amino acid sequence encoded by any one of SEQ ID NOs: 52-56.

15. An expression vector, **characterized in that**: it comprises a polynucleotide molecule encoding the anti-CD16a single-domain antibody according to any one of claims 1 to 4, or a polynucleotide molecule encoding the Fc-fusion antibody or the humanized antibody according to claim 5, or a polynucleotide molecule encoding the bispecific antibody or the multispecific antibody according to any one of claims 7-12, or the polynucleotide molecule according to claim 13 or claim 14.

16. A host cell, **characterized in that**: it can express the anti-CD16a single-domain antibody according to any one of claims 1 to 4, or the Fc-fusion antibody or the humanized antibody according to claim 5, or the bispecific antibody or the multispecific antibody according to any one of claims 7-12, or it comprises the expression vector according to claim 15.

17. A pharmaceutical composition, **characterized in that**: the pharmaceutical composition comprises the anti-CD16a single-domain antibody according to any one of claims 1 to 4 or the bispecific antibody or the multispecific antibody according to any one of claims 7-12, and a pharmaceutically acceptable carrier.

18. A medicament for use in treating a disease, **characterized in that**: it comprises the anti-CD16a single-domain antibody according to any one of claims 1 to 4 or the bispecific antibody or the multispecific antibody according to any one of claims 7-12 as an active ingredient.

19. Use of the anti-CD16a single-domain antibody according to any one of claims 1 to 4, or the bispecific antibody or the multispecific antibody according to any one of claims 7-12, or the pharmaceutical composition according to claim 17, in the manufacture of a medicament for treating a disease.

20. The use according to claim 19, **characterized in that**: the disease includes a tumor.
